# EUROPEAN PATENT SPECIFICATION

(11) **EP 1 007 559 B1**
(45) Date of publication and mention of the grant of the patent: **28.12.2005**
(21) Application number: 98945924.3
(22) Date of filing: 04.09.1998
(51) Int. Cl.: C07K 14/51, C07K 14/71, A61K 48/00

(54) **GENETICALLY ENGINEERED CELLS WHICH EXPRESS BONE MORPHOGENETIC PROTEINS**
GENETISCH MANIPULIERTE ZELLEN DIE KNOCHENMORPHOGENESEPROTEINEN EXPRIMIEREN
CELLULES GENETIQUEMENT MANIPULEES QUI EXPRIMENT DES PROTEINES MORPHOGENETIQUES D'OS

(30) Priority: 05.09.1997 US 57989 P; 04.09.1998 US 148234
(43) Date of publication of application: 14.06.2000
(73) Proprietor: Genetics Institute, LLC, Cambridge, MA 02140 (US); YISSUM RESEARCH DEVELOPMENT COMPANY OF THE HEBREW UNIVERSITY OF JERUSALEM, Jerusalem 91042 (IL)
(72) Inventor: MOUTSATSOS, Ioannis, Arlington, MA 02174 (US); GAZIT, Dan, 96920 Jerusalem (IL); ZILBERMAN, Yoram, 93781 Jerusalem (IL); TURGEMAN, Gadi, 97763 Jerusalem (IL)
(74) Representative: Frohwitter, Bernhard
(86) International application number: PCT/US1998/018603
(87) International publication number: WO 1999/011664

(56) References cited:
- WO-A-95/22611
- WO-A-96/39431
- LIEBERMAN J ET AL: "In vivo bone induction via retroviral gene transfer of BMP2 into a stromal cell line" TRANS ORTHOP RES SOC, vol. 43, 1997, page 223 XP002089721
- LIEBERMAN J ET AL: "Adenoviral gene transfer of recombinant BMP2 into human and rodent bone marrow cell induces bone formation in vivo." TRANS ORTHOP RES SOC, vol. 43, 1997, page 427 XP002089722
- LIEBERMAN J ET AL: "Regional gene therapy with a BMP-2-producing murine stromal cell line induces heterotopic and orthotopic bone formation in rodents" J ORTHOP RES , vol. 16, no. 3, May 1998, pages 330-339, XP002089723
- AHRENS M ET AL: "Expression of bone morphogenetic proteins-2 or -4 in murine mesenchymal progenitor C3H10T1/2 cells induces differentiation into distinct mesenchymal cell lineages" DNA CELL BIOL, vol. 12, no. 10, 1993, pages 871-880, XP002089724
- PROCKOP D J: "Marrow stromal cells as stem cells for nonhematopoietic tissues" SCIENCE, vol. 276, 4 April 1997, pages 71-74, XP002089725

## Description

### FIELD OF THE INVENTION

The present invention relates to methods of genetically engineering cells to produce cytokines. More specifically, the present invention relates to methods of transforming cells with cDNA encoding transforming growth factors of the TGF-β superfamily of proteins, which are useful for treatment of conditions such as osteoporosis and osteoarthritis.

### BACKGROUND OF THE INVENTION

During fracture repair, pluripotent stem cells (osteogenic progenitors) differentiate into osteoblasts and form callus. Bone morphogenetic proteins (BMPs) are known to initiate cartilage and bone progenitor cell differentiation and to induce bone formation. To date, there is no effective therapy for fractures that heal with difficulty (non-union fractures). Gene therapy with various cells treated with genes has been attempted. However, there is currently no known method by which cells which are potentially responsive to BMPs can be used for growth factor delivery to signaling receptors of transplanted cells (autocrine effect) and host progenitor stem cells (paracrine effect), for the engraftment, differentiation and stimulation of new bone growth.

### SUMMARY OF THE INVENTION

Accordingly, the present invention provides methods comprising transforming cells with cDNA encoding growth factors which are useful for treatment of conditions such as osteoporosis and osteoarthritis, as well as for treating fractures, particularly difficult to heal fractures, such as non-union fractures. In particular embodiments, the methods comprise transforming cells with cDNA encoding one or more factors from the transforming growth factor beta (TGF-β) superfamily of proteins. The TGF-β superfamily includes the bone morphogenetic proteins (BMPs), growth and differentiation factors (GDFs) and other structurally related proteins which are described in further detail herein. In other embodiments, the present invention comprises cells which have been transformed with cDNA encoding growth factors, such as proteins of the TGF-β superfamily, and methods of treating patients by implantation of such cells. The cells useful in the present invention may be human stem cells, as well as cultured cell lines and bone marrow stem cells. In the preferred embodiments, the cells have been transformed with cDNA encoding one or more BMPs or GDFs. In some preferred embodiments, the cells which serve as the host in the invention contain endogenous membrane bound receptors which are able to bind to BMPs or GDFs. Many such cell lines are known and are publicly available. These include, for example, U2-OS osteosarcoma. Other cell lines that are known to express BMP receptors may also be used. In other preferred embodiments, the cells contain endogenous membrane bound receptors which bind to proteins which have been implicated in bone, cartilage and/or other connective tissue formation. These include receptors for parathyroid hormone, parathyroid hormone related peptide, cadherins, activin, inhibin, hedgehog genes, IGF, Fibroblast Growth Factor and OGP.

In other preferred embodiments, the cells which serve as hosts may be transformed with DNA encoding both a growth factor, such as a BMP or a GDF, and a membrane bound receptor protein, such as a BMP receptor protein, other TGF-β receptor protein, parathyroid hormone receptor, cadherin receptor protein, or other related receptor protein. In a particular embodiment, the cells may be transformed with a DNA sequence encoding a truncated version of the growth factor and/or the membrane bound receptor protein. The truncated growth factor should preferably retain its biological activity, and the truncated receptor protein should preferably retain the ligand binding domain.

Suitable host cells for use in the present invention include cell lines and primary cells, as well as any cell which may be cultured and manipulated *in vitro* and/or *in vivo*, particularly for the introduction of several genes into the cells.

One of the advantages of the present system is that it takes advantage of both paracrine autocrine effects: e.g. the effects of the transformed factors on differentiation of the surrounding cellular environment, as well as the effects of the cellular environment on increasing expression of the transformed factors.

### BRIEF DESCRIPTION OF THE DRAWINGS

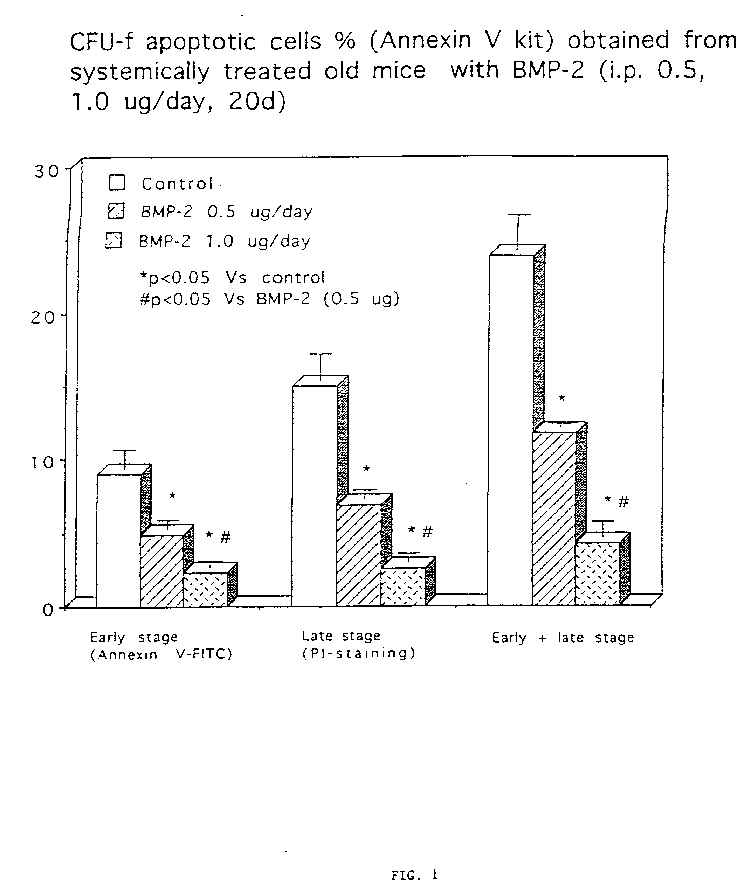

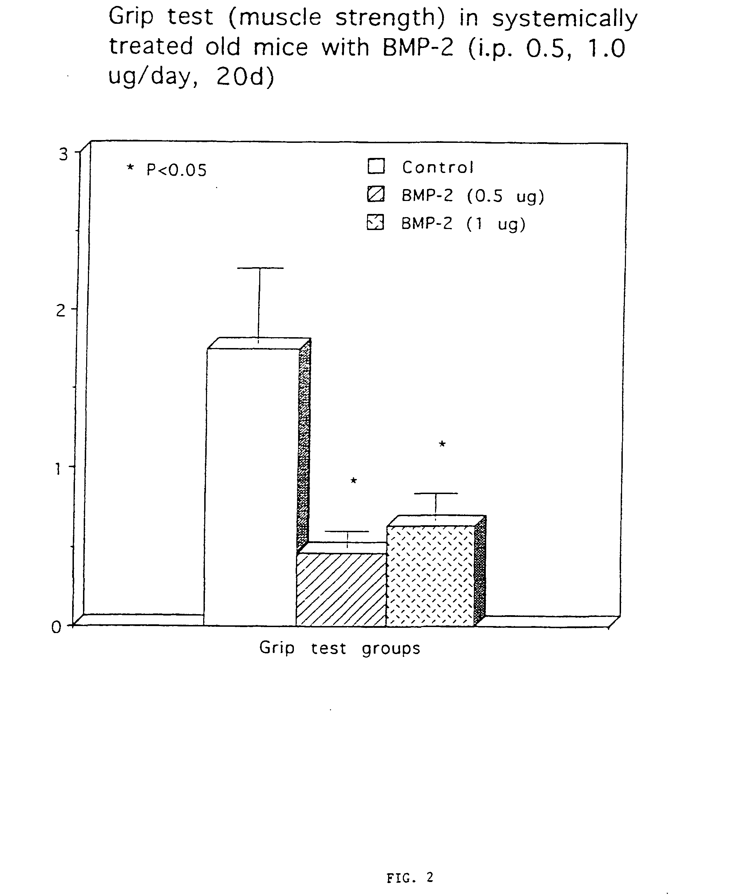

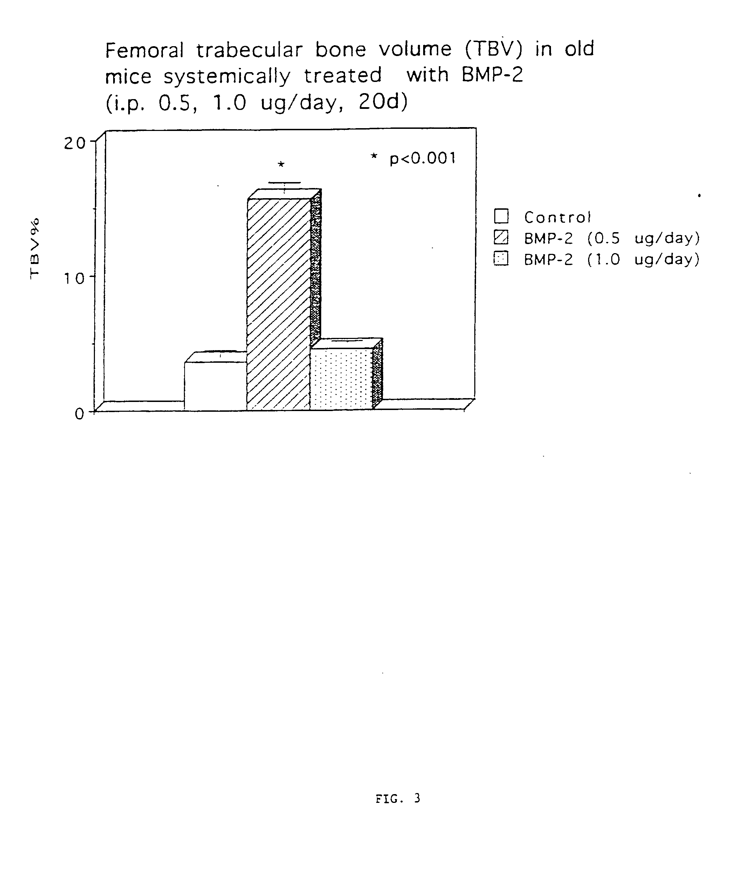

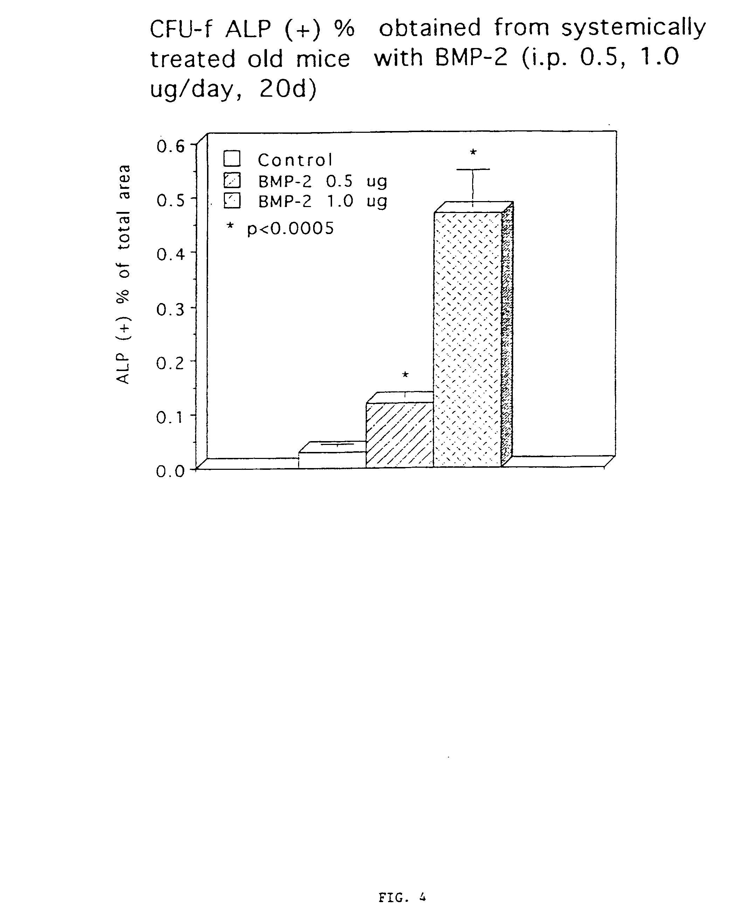

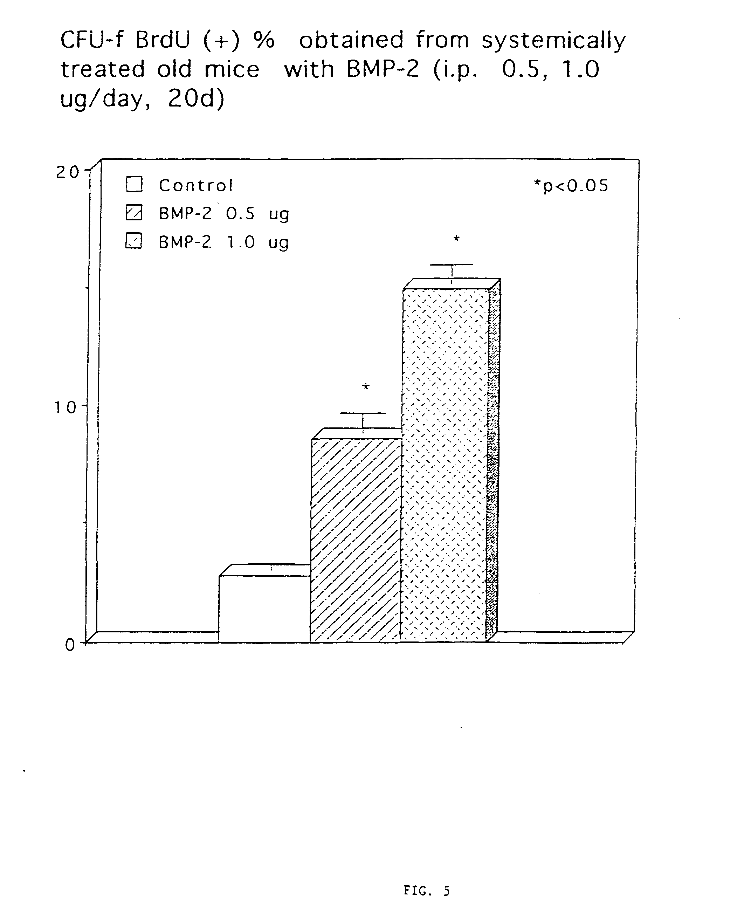

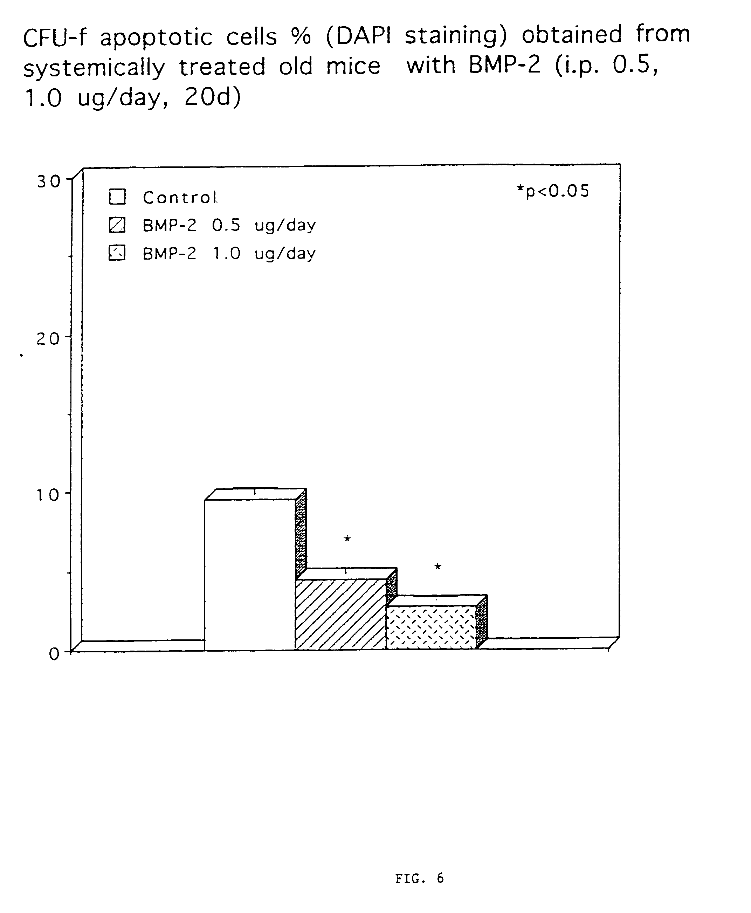

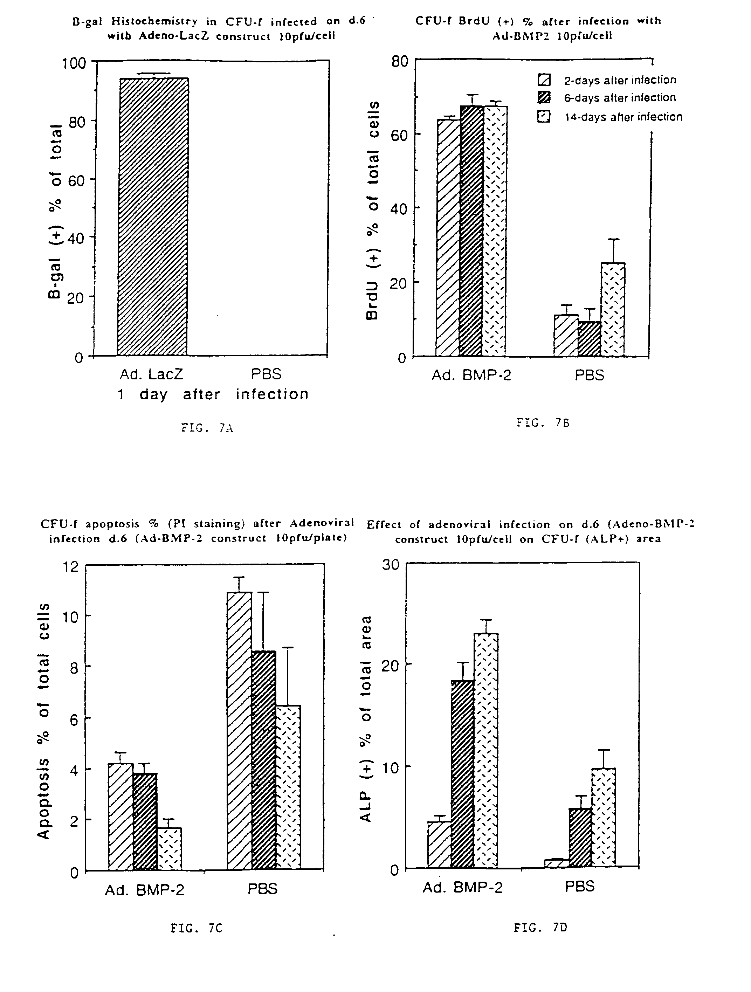

Figures 1 to 6 show results which demonstrate the dose-dependent effect of rhBMP-2 administered systemically on muscle strength, trabecular bone volume (TBV), CFU-f differentiation and cell characteristics. Old mice were treated with rhBMP-2 administered systemically [(i.p.).5, 1.0 µg/day, 20 d]. Figure 1 shows the results of a grip test of muscle strength. Figure 2 shows bone induction by femoral trabecular bone volume (TBV). Figure 3 shows the osteoblastic differentiation of CFU-f represented by alkaline phosphatase histochemistry (ALP). Figure 4 shows the cellular proliferation of CFU-f represented by BrdU. Figure 5 shows the cellular apoptosis of CFU-f represented by DAPI staining. Figure 6 shows the cellular apoptosis of CFU-f cells represented by Annexin V-FITC and PI-staining. Figure 7 shows the effect of BMP-2 by adenoviral infection: infection efficiency rate [Figure 7A]; increasing proliferation [Figure 7B]; decreasing apoptosis [Figure 7C]; and enhancing osteoblastic differentiation [Figure 7D].

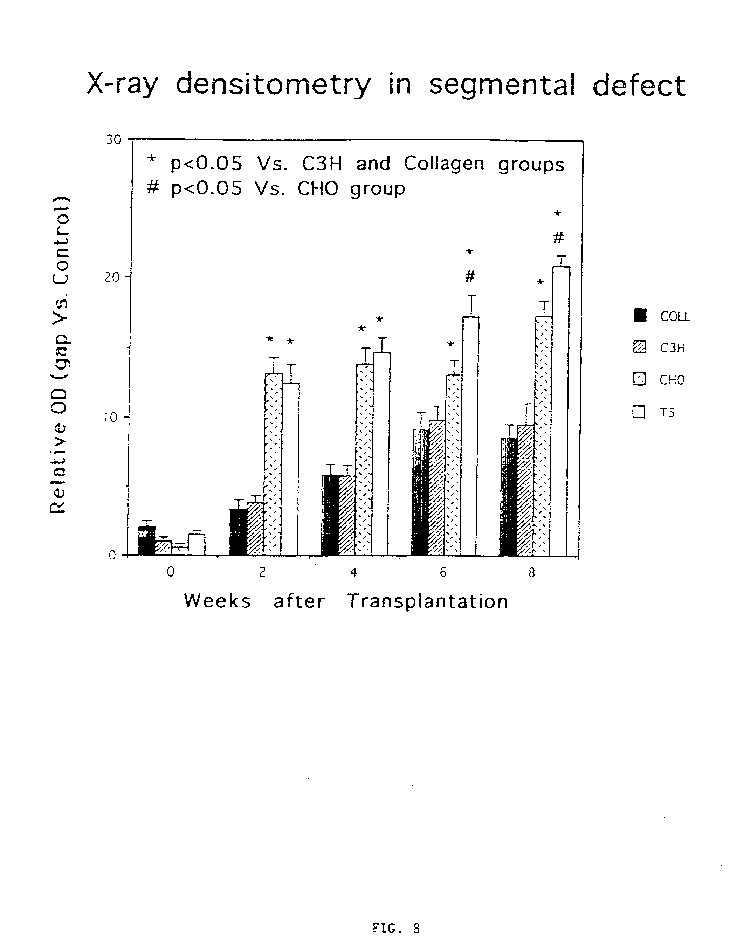

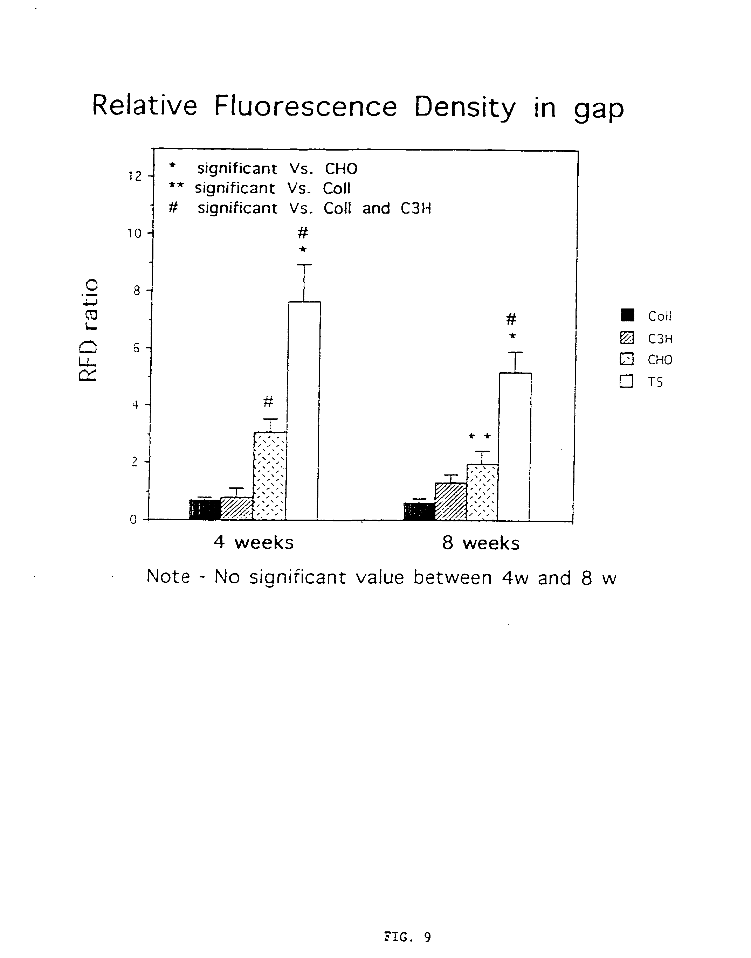

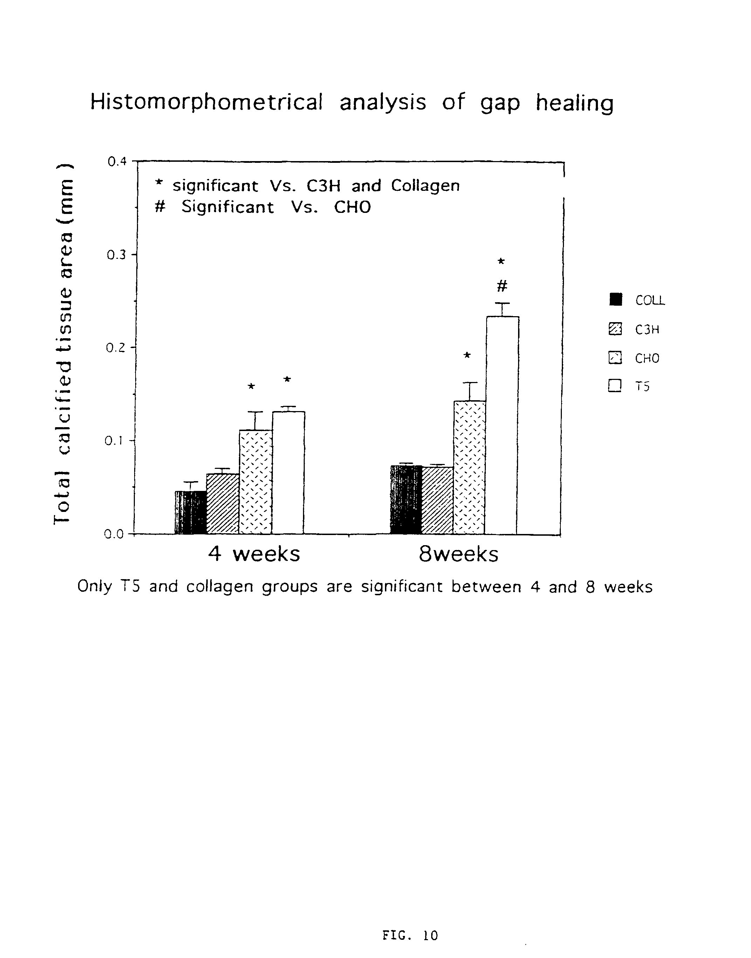

Figures 8 to 10 show the densitometry fluorescence density and histomorphometric analyses of gaps filled with BMP-2 soaked collagen sponge, C3H, CHO and T5 cell lines. Figure 8 shows the X-ray densitometry in segmental defects. Figure 9 shows the relative fluorescence density. Figure 10 shows the total calcified tissue area.

### DETAILED DESCRIPTION OF THE INVENTION

Among the DNA molecules useful in the present invention are those comprising the coding sequences for one or more of the BMP proteins BMP-2. BMP-3, BMP-4, BMP-5, BMP-6 and BMP-7, disclosed for instance in United States Patents 5,108,922; 5,013,649; 5,116,738; 5,106,748; 5,187,076; and 5,141,905; BMP-8, disclosed in PCT publication WO91/18098; and BMP-9, disclosed in PCT publication WO93/00432, BMP-10, disclosed in PCT application WO94/26893; BMP-11, disclosed in PCT application WO94/26892, or BMP-12 or BMP-13, disclosed in PCT application WO95/16035, or BMP-15, disclosed in PCT application WO96/36710 or BMP-16, disclosed in co-pending patent application serial number 08/715/202, filed September 18, 1996.

Other DNA molecules which may also be useful include those encoding Vgr-2, and any of the growth and differentiation factors [GDFs], including those described in PCT applications WO94/15965; WO94/15949; WO95/01801; WO95/01802; WO94/21681; WO94/15966; and others. Also useful in the present invention may be BIP, disclosed in WO94/01557; and MP52, disclosed in PCT application WO93/16099.

Other DNA molecules which may be useful, in addition to DNA encoding a BMP protein, include DNA molecules encoding other therapeutically useful agents including growth factors such as epidermal growth factor (EGF), fibroblast growth factor (FGF), transforming growth factor (TGF-α and TGF-β), *hedgehog* proteins such as sonic, indian and desert *hedgehog*, parathyroid hormone and parathyroid hormone related peptide, cadherins, activins, inhibins, and IGF, FSH, *frizzled, frzb* or *frazzled* proteins, PDGF and other endothelial growth factors, BMP binding proteins such as chordin and fetuin, estrogen and other steroids as well as truncated versions thereof, and transcription factors such as *wnt* proteins, mad genes and cbfa.

Among the receptors which may be useful for cotransfection in the present invention, are the various known BMP and TGF-β receptors, such as ALK-1 through ALK-6, and their species counterparts, particularly human, as well as receptors for parathyroid hormone, parathyroid hormone related peptide, cadherins, activin, inhibin, hedgehog genes, IGF, FGF, OGP, PDGF, endothelial growth factors, *frizzled* proteins, estrogen, follicle stimulating hormone and other steroid receptors. Thus, the host cell may be transformed with one or more DNA sequences encoding such a receptor protein. In a particular embodiment, the cell may be transformed with one or more DNA sequences encoding a truncated form of the above receptor proteins. It is preferred that the truncated form retain the ligand binding domain, but exclude the membrane bound domain, resulting in the expression of a secreted receptor protein.

In a preferred embodiment, the cells which are transformed are cultured cell lines, although primary cells may also be used. Cell lines may have particularly advantages in that they are easy to manipulate *in vitro,* particularly for the introduction of several genes into the cells. Cell lines are also advantageous in that they grow relatively rapidly and are relatively easy to achieve high cell number. In a particular embodiment, the cell lines may be coated with alginate or other suitable materials, or may otherwise have their antigenicity blocked, in order to reduce or avoid reaction with T cells. Among the human cell lines which contain BMP receptors, and which may be preferred for use as host cells in the present invention are TIG-3-20 (lung fibroblast), SF-TY (skin fibroblast), HUO-3N 1 (osteosarcoma), NB-1 (neuroblastoma), HepG2 (hepatocarcinoma), NC65 (kidney adenocarcinoma), TMK-1 (stomach adenocarcinoma), PC3 (prostate adenocarcinoma), ABC-1 (lung adenocarcinoma), COLO201 (colon adenocarcinoma)[Iwasaki et al. J. Biol. Chem., 270:5476(1995)]; U2-OS osteosarcoma (Lind et al., Bone 18:53 (1996)); NG108-15 (neuroblastoma) (Perides et al. J. Biol. Chem. 269:765 (1994)); HOBIT (osteoblastic)(Zheng et al., J. Cell Physiol. 159:76 (1994)); Saos-2 and HOS (osteosarcomas), HaCaT (keratinocyte)(Nissinen et al., Exp. Cell Res., 230:377 (1997)); AG1518 (foreskin fibroblast) and Tera-2 (teratocarcinoma)(ten Dijke et al., J. Biol. Chem. 269:16985(1994)); TE85 (osteosarcoma)(Malpe et al., BBRC 201:1140 (1994)); and HepG2 (hepatocarcinoma)(Song et al., Endocrin. 136:4293 (1995)). Human primary cells which have been shown to have BMP receptors, and which may be preferred for use as host cells in the present invention include bone marrow cells (Cheng et al., Endocrin. 134:277 (1994)); osteoblasts (Lind et al., Bone 18:53 (1996)); ligament cells (Kon et al., Calcif. Tissue Int., 60:291 (1997)); embryonic cells and keratinocytes (Nissinen et al., Exp. Cell Res., 230:377 (1997)); monocytes, neutrophils and fibroblasts (Postlethwaite et al., J. Cell Physiol. 161:562 (1994), Cunningham et al., PNAS 89:11740 (1992)); and hepatocytes (Song et al., Endocrin. 136:4293 (1995)). In addition, many other human and non-human cell lines and primary cells are known and can be used in the present invention. For veterinary purposes, cell lines and primary cells of the same species are preferred.

In the present invention, the vectors used for incorporation and expression of the DNA are preferably viral in origin, particularly adenoviruses, as well as retroviruses. Adenoviruses are advantageous in that they do not require cells in the state of proliferation, and have a high efficiency rate of infection both *in vitro* and *in vivo*, whereas retroviruses are more often suitable for *in vitro* infection. Adenoviruses also offer high levels of transgene expression and the ability to achieve high titers. These advantages make adenoviruses more suitable for primary cells, cell lines and direct *in vivo* transduction. In addition, expression of the transgene is transient and the adenoviral vector does not integrate into the cell genome, making the vectors safer for use. All generations of recombinant adenoviruses are suitable, including the present generation, (E1 deleted), and new generations which have reduced antigenicity (E1, E3, E4 deleted viruses, or E1, E4 deleted and E3 overexpressed), Smith (1995); Dunbar (1996); Roemer (1992); Graham (1991); Kozarsky (1993); and Ilan (1997).

The expression of the genes which are expressed in the present invention may be constitutive or controlled. Controlling the expression can be achieved by external control by means of regulatory elements, such as with an inducibly controlled promoter, for example, a tetracycline controlled promoter, as further described herein, or by using regulatory elements from tissue specific or temporally specific genes to direct the expression only to certain specified differentiation pathways or at certain stages in differentiation. For example, the osteocalcin promoter may be used for induction at late stages of bone formation and calcification.

The methods of the present invention may be useful for the regeneration of tissue of various types, including bone, cartilage, tendon, ligament, muscle, skin, and other connective tissue, as well as nerve, cardiac, liver, lung, kidney, pancreas, brain, and other organ tissues. In addition, the methods of the present invention could be used to induce differentiation and/or regeneration of other tissue types, including at the embryonic level in the induction of epidermal, endodermal and mesodermal development.

In some embodiments, the cells of the present invention may be administered in combination with an appropriate matrix, for instance, for supporting the composition and providing a surface for bone, cartilage, muscle, nerve, epidermis and/or other connective tissue growth. The matrix may be in the form of traditional matrix biomaterials. The matrix may provide slow release of the expressed protein and differentiated cells and/or the appropriate environment for presentation thereof. In some embodiments, various collagenous and non-collagenous proteins are expected to be upregulated and secreted from the pluripotent stem cells. This phenomenon accelerates tissue regeneration by enhancing matrix deposition. Matrix proteins can also be expressed in the genetically engineered cells and enhance the engraftment and attachment of transplanted cells into the transplant area. For example, expression of integrin proteins or actin filament proteins may assist in such engraftment. Jones (1996).

The choice of matrix material is based on biocompatibility, biodegradability, mechanical properties, cosmetic appearance and interface properties. The particular application of the cellular based compositions will define the appropriate formulation. Potential matrices for the compositions may be biodegradable and chemically defined calcium sulfate, tricalcium phosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above mentioned types of material, such as polylactic acid and hydroxyapatite or coilagen and tricalcium phosphate. The bioceramics may be altered in composition, such as in calcium-aluminate-phosphate and processing to alter pore size, particle size, particle shape, and biodegradability.

The invention, in certain of its embodiments, is illustrated by the examples below. These examples are not limiting. As will be appreciated by those skilled in the art, many variations and combinations of the following examples are available. These combinations and variations constitute a part of the present invention.

### EXAMPLES

### Example 1: In Vivo Expression of BMP-2 in C.9 Cells Under Inducible Promoter

C3HBAGα cells were generated by infecting C3H10T1/2 cells with BAGα retrovirus encoding β-galactosidase.

In order to test the effects of regulated expression of BMP-2 under the control of Tet inducible promoter, by tetracycline, on C.9 cells *in vivo*, C.9 cells were transformed with a vector in which the cDNA for BMP-2 was expressed under the control of the Tet inducible promoter, and the C.9 cells were transplanted into the abdominal muscle, a non-regenerative site. Cells were localized in the muscle by X-gal histochemical staining, after 10, 21 and 31 days (frozen sections). Doxycycline (Dox) was used as tetracycline analog was administered P.O. No cyclosporine or other immunosuppresive drugs were administered.

### Results

C.9 transplants in the muscle developed into newly formed ectopic bone and cartilage, in (-Dox) animals (no systemic Dox treatment). Bone collar and cartilage were found in the transplant (day 10), distant from the host muscle. On day 21, prominent trabecular bone, cartilage, and bone marrow were found distant and adjacent to the host muscle. On day 31, prominent trabecular bone (no cartilage) was found in the center of the transplant. In (+Dox) animals (systemically treated with Dox), only mesenchymal (connective) tissue was formed in the transplants (bone and cartilage were not found). Transplant size and radiopacity was greater in (-Dox) animals compared to (+Dox) animals.

β-gal positive cells were found in transplanted cells, forming bone and cartilage (donor origin), in (-Dox) mice. On day 11, the highest number of β-gal positive cells were found, localized to the transplant newly formed bone (osteoblasts), cartilage (chondrocytes) and surrounding mesenchymal tissue. On day 21, β-gal positive cells were localized to new formed bone trabeculas (osteoblasts) and to hypertrophic cartilage (chondrocytes). After 31 days, no positive cells were observed. In (+Dox) animals, β-gal positive cells were not detected in (+Dox) animals.

### Conclusions

A "reciprocal differentiation system" is highly effective not only in a regenerating site (segmental defect), but in a non-regenerating site as well, for example, i.m. (*intra* muscular) transplantations of pluripotent stem cells overexpressing BMP-2 (inducible expression). The combination of pluripotent stem cells and BMP-2 expression (reciprocal differentiation system composed of autocrine and paracrine effects of BMPs) enhances a significantly differentiation process in the transplanted pluripotent stem cells. Utilizing this system, transplanted cells differentiate into bone and cartilage (as shown with β-gal expression). The system described has the advantage that BMP-2 protein is being induced *in vivo*, delivers the gene of interest (for gene therapy purposes), and enables pluripotent stem cells to differentiate in the required direction (in regenerating and non-regenerating sites). Such a reciprocal differentiation system, having enhanced differentiation potential of pluripotent stem cells, is an effective and reliable system to enable the identification of novel biological activities of both novel and known cytokines.

### Example 2: In Vitro and In Vivo Expression of BMP-2 in C.9 Cells

In order to test regulated expression of BMP-2 under the control of Tet inducible promoter, by tetracycline, and its effect on C.9 cells both *in vitro* and *in vivo.* C.9 cells were generated by transfection of C3HBAGα cells with rhBMP-2 construct containing a tet regulated promoter. β-gal expression *in vitro* was determined by X-gal histochemical staining and immunofluorescence. BMP-2 expression *in vitro* was determined by immunohistochemistry.

C.9 cells were transplanted into a 3 mm segmental defect. Cells were localized in the gab by X-gal histochemical staining, after one week. C.9 cells were also transplanted into the abdominal muscle (non-regenerating site). Cells were localized in the muscle by X-gal histochemical staining, after 10 days (frozen sections). Doxycycline (Dox) was used as tetracycline analog for administration *in vitro* and *in vivo* (i.p. injections and oral administration).

### Results:

β-gal expression *in vitro* was shown to be non affected by Dox treatment, *in vitro.* Approximately 50% of the cells express β-gal. BMP-2 expression, *in vitro*, was shown to be regulated by Dox treatment. C.9 cells were shown to survive better in the segmental defect gap without the presence of Dox. C.9 transplants in the muscle were able to develop into newly formed ectopic bone, without the treatment of Dox. With treatment of Dox only, mild connective tissue was formed without any signs of bone formation. β-gal positive cells were found in transplant area (including bone particles) only in the absence of Dox. No positive cells were detected in the transplant in the presence of Dox.

### Conclusions

Doxycycline can regulate BMP-2 expression *in vitro* and affect C.9 cells' survival and bone induction *in vivo.*

### Example 3: In Vivo Transplantations of T5 Cells

In order to test the hypothesis that T5 cells can survive, produce BMP-2 and differentiate into osteoblasts *in vivo*, resulting in increased healing of bone segmental defects, T5 cells were mounted on collagen sponges and transplanted into segmental defects (2.5 mm, 3 mm and 3.5 mm) in C3H mice radius. (C3H10T1/2 BAGα and C3H10T1/2 WT, collagen only and segmental defect only served as negative controls. Recombinantly produced human BMP-2 protein (3-10 µg) served as positive control). T5 (and C3h BAGα) cells were localized *in vivo* by X-gal histochemical staining for β-gal (frozen sections). β-gal and BMP-2 expression were co-localized by β-gal histochemical staining done first, and BMP-2 immunohistochemical staining done second, or by double immunofluorescence (frozen sections). Fracture healing was assessed by histology, X-ray photographs, computerized X-ray densitometry and computerized fluorescence densitometry.

### Results

T5 cell transplants have shown an increased radiopacity in X-rays from two weeks onwards and even bridging of the defect at 6 weeks. T5 cells have been localized to the gap area at different times, in the transplanted sponge and on later newly-formed bone and osteoprogenitor cells. T5 cells have also been shown to produce BMP-2 *in vivo.* Negative control groups show lack of healing (collagen only and segmental defect only, or reduced healing in C3H BAGα and WT compared to T5 cells).

BMP-2 (protein/sponge) implants formed bone already at two weeks after implantation. The new bone was comprised of bone trabeculas and fatty bone marrow. X-ray and fluorescence computerized densitometry demonstrate quantitatively the results mentioned in sections above.

### Conclusions

The ability of T5 cells both to produce BMP-2 and to differentiate (*in vitro*) and localize to a newly formed bone (*in vivo*), correlate with the increased ability of T5 transplant to heal segmental defects.

### Example 4: Adenoviral and Retroviral Infection of Primary Culture

In order to test the efficiency of gene delivery into marrow osteoprogenitor cells. Preliminary experiments were conducted with adeno/retro viruses with the LacZ construct. Marrow osteoprogenitor cells were grown, *ex vivo* (in cfu-f culture). Cultures were infected (*in vitro*) with recombinant retrovirus BAGα encoding LacZ (β-gal) gene, and adenoviral E1-LacZ. Infected cells were detected with X-gal histochemical staining for β-gal.

### Results

High rates of infections were achieved in both adeno and retroviral infections. Sixty-five to ninety per cent [65-90%] of the cells have expressed β-gal.

### Conclusions

The above experiments demonstrated that marrow osteoprogenitor cells can be genetically modified to express genes, and can be utilized in gene therapy in bone. Various genes can be expressed, among them cytokines and growth factors such as bone morphogenetic proteins (BMPs), growth and differentiation factors (GDFs) and other members of the transforming growth factor beta (TGF-β) superfamily of proteins. Such genes may be delivered by retrovirus *ex vivo* or by adenovirus for *ex vivo* or *in vivo* transformation.

### Example 5: Autocrine Activity in Reciprocal Differentiation System:

10T cells were transformed with DNA encoding BMP-2 and parathyroid hormone receptor (PTHR). Several implantations were completed which indicated that 10T overexpressing BMP-2 make cartilage and bone. However, cells overexpressing both BMP-2 and PTHR evidenced only cartilage formation with no bone formation observed. This cartilage formation is believed to be due to the effects of BMP-2 in influencing the binding of parathyroid hormone to its receptor, thus an autocrine effect. The cells may similarly be manipulated to express inducible BMP-2 receptors. In such a system, the autocrine activity of such cells can be dramatically altered and/or controlled to exert a desired biological effect.

### Example 6: Systemic Effects of BMP-2 in Adult Osteoporotic Mice

In order to test the *in vivo* effect of BMP-2 on bone marrow osteoprogenitor cells (CFU-f), trabecular bone compartment and physical ability in osteoporotic mice, 24 month old BALB/c male mice received systemic administration of rhBMP-2 at 0.5 µg/mouse/day i.p. for 20 days. A control group was injected with 200 µl BSA/PBS 0.1%. The mice were labeled with Calcein Green (2.5 mg/kg) seven days and two days before sacrifice for fluorescent bone morphometry. Bone histomorphometry of tibia and femurs is performed using plastic and paraffin histological sections. Histology of internal organs, including liver, spleen, kidney and testis, is performed by Paraffin sections (H&E staining). Psychobiology assays for the determination of physical ability, behavior and activity is performed using computerized systems with video monitoring in order to monitor a Grip Test. Open Field and Water-Maze Test.

### Results:

In Figures 1 to 6, results are shown which demonstrate the dose dependent effect of rhBMP-2 administered systemically on muscle strength, trabecular bone volume, CFU-f differentiation and cell characteristics. Internal organs were not affected. However, increased testicular spermatogenesis was noted.

The "Grip" test revealed significant diminution of time (about three-fold) in the BMP-2 treated mice. (See graph). The "Open Field" and "Water-Maze" tests did not reveal significant differences in mice behavior. The Grip test results demonstrate clearly that older osteoporotic mice systemically injected with rhBMP-2 show increased physical potency. This is the first indication that BMP-2 has systemic effect on muscles of old mice, a model for osteoporosis. These experiments exclude any negative systemic effect of BMP-2 on CNS (no adverse effect on behavior, memory *etc*.).

### Example 7: Adenoviral and Retroviral Infection of Primary Cultures

To test the *in vitro* effects of BMP-2 on primary cultures in the present invention, bone marrow stromal cells recovered from femur and tibia (CFU-f) were plated in MEM-α supplemented with 10% FCS and Pen/Strep 100 µ/ml in 35 mm plates at density 10⁶ cells/plate and infected with (1) BAGα retrovirus encoding LacZ gene; (2) adenovirus encoding LacZ; or (3) adenovirus encoding rhBMP-2. The transfected CFU-f cells were cultured *in vitro* for a 12 day period with changing of the medium and supplementation for mineralization twice a week. CFU-f was assayed for alkaline phosphatase histochemistry (ALP), proliferation (BrdU) and apoptosis (DAP1).

Retroviral infection achieved an infection efficiency rate of about 65-70%, and the adenovirus achieved more than 90% efficiency rate of infections. In addition, adenoviral infection with BMP-2 altered marrow stromal cell fate and cellular characteristics, by enhancing osteoblastic differentiation (ALP), increasing proliferation, and decreasing apoptosis [Figures 7A-D].

These experiments demonstrate that marrow stromal cells are suitable hosts for *in vitro* transfection with adenoviral vectors, and can serve as host cells for use in the reciprocal differentiation system of the present invention.

### Example 8: Autocrine/Paracrine System Effects Compared to Paracrine Effect

The following cell lines were transplanted into a radial segmental defect (2.5 mm) in mice: T5 (C3H10T1/2 cells coexpressing β-gal and rhBMP-2); C3H BAGα (C3H10T1/2 cells expressing only β-gal; and CHO cells overexpressing rhBMP-2. In addition, mice were transplanted with carrier only (collagen sponge) as a negative control.

In this system T5 cells represent both the paracrine and autocrine effect: CHO cells, which are not osteogenic, and cannot differentiate in the osteogenic pathway, represent the paracrine effect only. The paracrine effect can be estimated by rhBMP-2 secretion to the environment. It was found in *vitro* that T5 cells secrete 5 ng active rhBMP-2/day/10⁷ cells, and CHO cells secrete 840 ng active rhBMP-2/day/10⁷ cells, meaning that CHO cells secrete 160 times more BMP-2 than T5 cells, and therefore have greater paracrine effects than T5 cells.

The quantitative results of the gap healing represented in X-ray densitometry, fluorescence and morphometry graphs, clearly demonstrated that T5 cells had higher scores in all parameters than CHO cells after 6-8 weeks, and thus had a greater therapeutic potential than CHO [Figures 8 to 10]. The superior results obtained by T5 cells cannot be attributed to the paracrine effect only, since CHO cells have significantly higher paracrine effect potential than T5 cells. Therefore, it is concluded that the autocrine effect of rhBMP-2 expression on T5 cells themselves played a significant role in these results. T5 cells were shown *in vitro* to differentiate spontaneously to osteoblasts: *in vivo,* they were shown to express rhBMP-2 and display the morphology of differentiated osteoblasts (double immunofluorescence).

Additional evidence of the importance of the autocrine effect is demonstrated by transplantations of two cell lines (C3H10T1/2) cells which express rhBMP-2 in the same manner, however one of the cell lines is additionally transfected to overexpress the PTH/PTHrP receptor. Overexpression of PTH/PTHrP receptor inhibited the autocrine effect of late stages of differentiation of the cells and therefore represents primarily the paracrine effect. Upon muscle transplantations *in vivo,* heterolopic excess cartilage and bone are formed in the cell which expresses rhBMP-2 only. However, in the cell which expresses both rhBMP-2 and PTH/PTHrP receptor, only dense connective tissue and small islands of cartilage were formed. Since the paracrine effect of these two cell lines is expected to be the same, it is the difference in autocrine effects which is primarily responsible for the altered results in bone formation and differentiation.

The most important advantage of combined paracrine and autocrine effects is the introduction of the responsive elements, i.e., the cells themselves, to the area in which the desired transgenic protein is being produced. All therapeutic proteins exert their effect on target cells which respond to them, and initiate a biological effect. BMPs and other bone inductive growth factors act, primarily on stromal progenitor cells present in the bone marrow environment. In order to exhibit an effective paracrine effect with these proteins, the presence of significant amounts of osteoprogenitor cells is required. However, in large segmental defects, significant mass of bone is deficient, as well as in osteoporosis, in which bone lacks stem cells (Kahn 1995). In these indications, it has been shown that the ability to respond to BMP and other bone growth factors is reduced because of the reduced number or responsiveness of stem cells to the osteoinductive proteins. Fleet et al., Endocrinology, 137:4605-4610 (1996). Accordingly, the advantages of the reciprocal differentiation system described herein lies in the combined paracrine and autocrine effects which allow the genetically modified cells to participate actively in the healing and regeneration processes.

### Example 9: In Vitro and In Vivo Effects

### In Vivo:

### A. Cell lines:

(1) C3H BAGα cells were generated by infecting C3H10T1/2 cells with BAGα retrovirus encoding β-galactosidase.
(2) T5 (T5-B2C-BAP) cells were generated by transfected of C3H10T1/2 cells with rhBMP-2 construct, encoding human BMP-2 cDNA under the control of SV40 promoter, and further infection with BAGα retrovirus encoding β-galactosidase.
(3) CHO-rhBMP-2 cells were generated by transfecting CHO (DUKX) cells with rhBMP-2 construct only. Cells were grown in DMEM supplemented with 10% fetal calf serum, 2 mM L-glutamate and 100 units/ml penicillin and streptomycin.

### B. Differentiation Assays:

(1) Alkaline phosphatase for osteoblastic phenotype, Oil red O and Alcian blue for fat and cartilage phenotypes.
(2) BMP-2 expression was determined by northern blot. Immunohistochemistry and bioassay using W-20-17 cells.
(3) Co-localization of BMP-2 and β-gal was demonstrated by double immunofluorescence.

### In Vivo Effects

A. 10° cells from each cell line were mounted on collagen sponge and transplanted into segmental defects (2.5 mm) in C3H/HeN Mice. 16 in each group. Another group of mice were transplanted with the collagen sponge carrier only. Three mice were implanted with 10 µg of rhBMP-2 as histological control.
B. T5 (and C3H BAGα) cells were localized *in vivo* by X-gal histochemical staining for β-gal (frozen sections).
C. β-gal and BMP-2 expression were co-localized by double immunofluorescence (frozen sections).
D. Fracture healing was assessed quantitatively by computerized X-ray densitometry, computerized fluorescence densitometry and histomorphometry.
E. Histology was evaluated by Masson Trichrom staining.

### Results

### In Vitro

A. rhBMP-2 expression in T5 cells was demonstrated by northern blot and Immunohistochemistry. Estimated amount of rhBMP-2 secretion (by W20 cells bioassay) was found to be 5 ± 2.3 ng/24hours/10⁷ cells in T5 cells and 841.3 ± 88 ng/24 hours/10⁷ cells in CHO rhBMP-2 cells.
B. T5 cells were shown to co-express BMP-2 and β-gal in cultures.
C. T5 cells were differentiated spontaneously into osteoblasts even without anv treatment, different from C3H BAGα cells which differentiated only in the presence of Ascorbate and BMP-2. No fat or cartilage phenotypes were found.
D. BMP-2 expression was found to be correlated with differentiation. T5 differentiate and express BMP-2 *in vitro*, C3H BAGα serving as control, do not express BMP-2 and do not differentiate.
E. β-gal expression was found in differentiating T5 cells expressing ALP.

### In Vivo

A. X-rays densitometry (mean gap density relative to the Ulna's mean density) as a parameter of healing, showed the highest values with T5 and CHO-rhBMP-2 groups compared to C3H BAGα and collagen only. T5 group values were significantly higher than CHO-rhBMP-2 group at six and eight weeks after transplantation. C3H10T1/2 WT and collagen only groups did not differ from each other at each time point. Significant increase in densitometry ratio was observed already after two weeks in all groups (except collagen only group), which increased with time to the highest values at six weeks (C3H BAGα and collagen) and eight weeks (T5 and CHO-rhBMP-2).
B. Fluorescence density (relative to constant area of the Ulna's cortex) revealed the highest rate with T5 group, statistically significant when compared to all groups at four weeks and eight weeks. CHO-rhBMP-2 had significantly higher values from collagen and C3H BAGα at four weeks, and from collagen only at eight weeks. Although it seems that there was a decrease in fluorescence ratio from four weeks to eight weeks in T5 and CHO-rhBMP-2 and the opposite in C3H BAGα, it was not statistically significant.
C. T5 and CHO-rhBMP-2 groups had the highest rate of calcified newly formed bone in the gap compared to C3H BAGα and collagen groups at four weeks and at eight weeks. T5 differed from CHO-rhBMP-2 only at eight weeks: collagen and C3H BAGα did not differ significantly. Only collagen and T5 groups were significantly higher in eight weeks compared to four weeks.
D. Histologically, in T5 groups new bone can be seen *de novo* in the transplantation area. In addition, healing progresses by organized enchondral bone formation surrounding the gap edges. All other groups lack any signs of *de novo* bone formation in transplantation area, and the cartilage response around the gap edge is disorganized and less calcified. In CHO-rhBMP-2 group, excessive ectopic bone is formed (in the surrounding muscles) which is resorbed later on.
E. T5 and C3H BAGα cells engraft and localize to the surrounding of the gap edges after transplantation; after four weeks, T5 cells display osteoblasts morphology and express β-gal and BMP-2. High dose of BMP-2 (10 µg) were able to bridge the defect after eight weeks, with excessive trabecular bone and fatty bone marrow. However, the new bone formed has not shown continuation with the original bone edges which remained intact.

### Example 10: W-20 Assay

### A. Description of W-20 cells

Use of the W-20 bone marrow stromal cells as an indicator cell line is based upon the conversion of these cells to osteoblast-like cells after treatment with a BMP protein [Thies et al, Journal of Bone and Mineral Research, 5:305 (1990); and Thies et al, Endocrinology, 130:1318 (1992)]. Specifically, W-20 cells are a clonal bone marrow stromal cell line derived from adult mice by researchers in the laboratory of Dr. D. Nathan. Children's Hospital. Boston, MA. Treatment of W-20 cells with certain BMP proteins results in (1) increased alkaline phosphatase production, (2) induction of PTH stimulated cAMP, and (3) induction of osteocalcin synthesis by the cells. While (1) and (2) represent characteristics associated with the osteoblast phenotype, the ability to synthesize osteocalcin is a phenotypic property only displayed by mature osteoblasts. Furthermore, to date we have observed conversion of W-20 stromal cells to osteoblast-like cells only upon treatment with BMPs. In this manner, the *in vitro* activities displayed by BMP treated W-20 cells correlate with the *in vivo* bone forming activity known for BMPs. Below two *in vitro* assays useful in comparison of BMP activities of novel osteoinductive molecules are described.

### B. W-20 Alkaline Phosphatase Assay Protocol

W-20 cells are plated into 96 well tissue culture plates at a density of 10,000 cells per well in 200 µl of media (DME with 10% heat inactivated fetal calf serum. 2 mM glutamine and 100 Units/ml penicillin + 100 µg/ml streptomycin. The cells are allowed to attach overnight in a 95% air, 5% CO₂ incubator at 37°C. The 200 µl of media is removed from each well with a multichannel pipettor and replaced with an equal volume of test sample delivered in DME with 10% heat inactivated fetal calf serum, 2 mM glutamine and 1% penicillin-streptomycin. Test substances are assayed in triplicate. The test samples and standards are allowed a 24 hour incubation period with the W-20 indicator cells. After the 24 hours, plates are removed from the 37°C incubator and the test media are removed from the cells. The W-20 cell layers are washed 3 times with 200 µl per well of calcium/magnesium free phosphate buffered saline and these washes are discarded. 50 µl of glass distilled water is added to each well and the assay plates are then placed on a dry ice/ethanol bath for quick freezing. Once frozen, the assay plates are removed from the dry ice/ethanol bath and thawed at 37°C. This step is repeated 2 more times for a total of 3 freeze-thaw procedures. Once complete, the membrane bound alkaline phosphatase is available for measurement. 50 µl of assay mix (50 mM glycine, 0.05% Triton X-100, 4 mM MgCl₂, 5 mM p-nitrophenol phosphate, pH = 10.3) is added to each assay well and the assay plates are then incubated for 30 minutes at 37°C in a shaking waterbath at 60 oscillations per minute. At the end of the 30 minute incubation, the reaction is stopped by adding 100 µl of 0.2 N NaOH to each well and placing the assay plates on ice. The spectrophotometric absorbance for each well is read at a wavelength of 405 nanometers. These values are then compared to known standards to give an estimate of the alkaline phosphatase activity in each sample. For example, using known amounts of p-nitrophenol phosphate, absorbance values are generated. This is shown in Table I.

**TABLE I**

| Absorbance Values for Known Standards of P-Nitrophenol Phosphate | |
|---|---|
| P-nitrophenol phosphate umoles | Mean absorbance (405 nm) |
| 0.000 | 0 |
| 0.006 | 0.261 +/- .024 |
| 0.012 | 0.521 +/- .031 |
| 0.018 | 0.797 +/- .063 |
| 0.024 | 1.074 +/- .061 |
| 0.030 | 1.305 +/- .083 |

Absorbance values for known amounts of BMPs can be determined and convened to µmoles of p-nitrophenol phosphate cleaved per unit time as shown in Table II.

**TABLE II**

| Alkaline Phosphatase Values for W-20 Cells Treating with BMP-2 | | |
|---|---|---|
| BMP-2 concentration ng/ml | Absorbance Reading 405 nmeters | umoles substrate per hour |
| 0 | 0.645 | 0.024 |
| 1.56 | 0.696 | 0.026 |
| 3.12 | 0.765 | 0.029 |
| 6.25 | 0.923 | 0.036 |
| 12.50 | 1.121 | 0.044 |
| 25.0 | 1.457 | 0.058 |
| 50.0 | 1.662 | 0.067 |
| 100.0 | 1.977 | 0.080 |

These values are then used to compare the activities of known amounts of BMP-16 to BMP-2.

### C. Osteocalcin RIA Protocol

W-20 cells are plated at 10⁶ cells per well in 24 well multiwell tissue culture dishes in 2 mls of DME containing 10% heat inactivated fetal calf serum. 2 mM glutamine. The cells are allowed to attach overnight in an atmosphere of 95% air 5% CO₂ at 37°C. The next day the medium is changed to DME containing 10% fetal calf serum, 2 mM glutamine and the test substance in a total volume of 2 ml. Each test substance is administered to triplicate wells. The test substances are incubated with the W-20 cells for a total of 96 hours with replacement at 48 hours by the same test medias. At the end of 96 hours. 50 µl of the test media is removed from each well and assayed for osleocalcin production using a radioimmunoassay for mouse osteocalcin. The details of the assay are described in the kit manufactured by Biomedical Technologies Inc., 378 Page Street, Stoughton, MA 02072. Reagents for the assay are found as product numbers BT-431 (mouse osteocalcin standard). BT-432 (Goat anti-mouse Osteocalcin), BT-431R (iodinated mouse osteocalcin), BT-415 (normal goat serum) and BT-414 (donkey anti goat IgG). The RIA for osteocalcin synthesized by W-20 cells in response to BMP treatment is carried out as described in the protocol provided by the manufacturer.

The values obtained for the test samples are compared to values for known standards of mouse osteocalcin and to the amount of osteocalcin produced by W-20 cells in response to challenge with known amounts of BMP-2. The values for BMP-2 induced osteocalcin synthesis by W-20 cells is shown in Table III.

**TABLE III**

| Osteocalcin Synthesis by W-20 Cells | |
|---|---|
| BMP-2 Concentration ng/ml | Osteocalcin Synthesis ng/well |
| 0 | 0.8 |
| 2 | 0.9 |
| 4 | 0.8 |
| 8 | 2.2 |
| 16 | 2.7 |
| 31 | 3.2 |
| 62 | 5.1 |
| 125 | 6.5 |
| 250 | 8.2 |
| 500 | 9.4 |
| 1000 | 10.0 |

### EXAMPLE 11: Engineered pluripotent progenitor cells integrate and differentiate in regenerating bone: a novel regional cell-mediated gene therapy

Among the approximately 6.5 million fractures suffered in the United States every year, about 20% are difficult to heal. As yet, for most of these difficult cases there is no effective therapy. We have developed a mouse radial segmental defect as a model experimental system for testing the capacity of genetically engineered pluripotent progenitor cells (C3H10T1/2 clone expressing rhBMP-2), for gene delivery, engraftment, and induction of bone growth in regenerating bone. Transfected progenitor cells expressing rhBMP-2 were further infected with a vector carrying the *LacZ* gene, that encodes for β-galactosidase (β-gal). *In vitro* levels of rhBMP-2 expression and function were confirmed by immunohistochemistry, and bioassay. *In vitro*, progenitor cells spontaneously differentiated into osteogenic cells expressing alkaline phosphatase. Progenitor cells were transplanted *in vivo* into a radial segmental defect (regenerating site). Engrafted progenitor cells were quantitatively localized *in vivo* by β-gal expression, and immunohistochemical assays revealed that engrafted cells that had differentiated into osteoblasts and co-expressed β-gal and rhBMP-2. The main control groups included *lacZ* clones of WT-C3H10T1/2-LacZ, and CHO-rhBMP-2 cells. New bone formation was measured quantitatively *via* fluorescent labeling, which revealed that at 4-8 week post-transplantation. GEPMSC significantly (P<0.01) enhanced segmental defect repair. The present study shows that cell-mediated gene transfer is useful for delivery to signaling receptors of transplanted cells (autocrine effect) and host progenitor cells (paracrine effect), suggesting the ability of progenitor cells to engraft, differentiate, and stimulate bone growth. Thus, gene therapies may be useful for non-union fractures which do not otherwise heal in humans.

### INTRODUCTION

It is known that non-union radial fractures be can healed by increasing the local concentration of a signal molecule (like BMP-2) for osteogenesis and bone formation. In the present experiments, the inventors demonstrate that a protein may be delivered by progenitor cells genetically engineered to express the transgene for this signal molecule. Recombinant human BMP-2 (rhBMP-2) has been shown to be a highly osteoinductive protein that induces *in vitro* osteogenic differentiation in several progenitor cell types and can induce *in vivo* bone formation in ectopic sites as well as in non-union fractures. A model system was created using non-union radial fractures in mice as a model for bone fractures that will not heal under normal conditions, to allow measurement of new cartilage and bone tissue formation in these large bone defects induced by the presence of progenitor cells (C3H10T1/2) genetically engineered to express rhBMP-2 (C3H-BMP2). The cells were transplanted on collagen sponges (see Methods section) which were placed surgically into the radial bone fracture (2.5 mm segmental defect) created in female C3H/HeN mice. Four control groups were used. In all cases a single type of mouse (C3H/HeN) was used. In the experiment a group was treated by implanting a collagen sponge carrying one of the following: I) an aliquot of 10⁶ C3H-BMP2 cells; ii) an aliquot of 10⁶ genetically engineered non-progenitor cells (CHO-BMP2); iii) an aliquot of 10⁶ progenitor cells which had not been genetically engineered (C3H-WT); iv) no cells at all: v) no cells but on which were placed 3 ug of rhBMP-2. This last control group was a positive control, as the protein has previously been described in the literature.

There are many well known orthopedic techniques for the treatment of bone fractures. Among these, protein therapy is well known but is not yet commonly used. The important difference between the present system we are describing here and more standard protein therapy is that the protein is delivered to the *locus* by cells carrying the gene for the desired protein as a transgene. Since the cells have been engrafted into the diseased host tissue, the expression of the transgene creates a supply of the therapeutic protein in the vicinity of the lesion to be healed. Cells for this purpose are chosen primarily for their ability to provide long-term stable expression of the transgene in question. Thus, the cells to be engrafted must be characterized by long-term survival and by the ability to stably integrate into host tissue.

In CNS, following *in vivo* transplantation to host tissues, undifferentiated pluripotent progenitor cells integrate and differentiate successfully. Thus, in CNS, undifferentiated pluripotent progenitor cells are suitable candidates for use in cell mediated gene therapy. The long-term survival and successful integration of progenitor cells into host tissue makes them particularly appropriate for the case of tissue repair. By using undifferentiated pluripotent progenitor cells, it is believed that efficient transgene expression in the damaged tissue (paracrine mechanism) is increased, while maintaining the transgene effect on the progenitor cells themselves (autocrine mechanism). In addition, progenitor cells can communicate with the host tissue *via* their own signal molecules, as well as the signal molecules of the host cells which can affect the engrafting cells. Neuronal progenitor cells have previously been used to repair central nervous system dysfunction: they have been shown to integrate efficiently into the cytoarchitecture of the host central nervous system (CNS) and to permit the stable expression of the transgenes. Moreover, as has been demonstrated in CNS, progenitor cells themselves have the potential to actively participate in the healing process. These results suggest that progenitor cells can serve not only as a vehicle for transgene expression, but can themselves participate in the repair process and become an integral part of the host tissue. Moreover, it is believed that progenitor cells themselves can be affected by expression of the transgenes that they are carrying (autocrine mechanism). It is also believed that there is an increase in the engraftment, differentiation and therapeutic potential of such progenitor cells, and that other non-progenitor cells, like fibroblasts, lack the autocrine mechanism, and so will presumably have lesser therapeutic effects, compared to progenitor cells. Thus, the progenitor cells may have a specific advantage over other cell types in cell-mediated gene therapy for tissue repair.

### RESULTS

### Generation and characterization of genetically engineered progenitor cells

We generated genetically engineered progenitor cells from the C3H10T1/2 pluripotent progenitor cell line capable of differentiating into myogenic, osteogenic, chondrogenic, or adipogenic cell lines. C3H10T1/2 cells were transfected by plasmid pED4 that encodes a bicistronic transcript having the configuration of rhBMP-2cDNA-EMC leader sequence-neoR under the control of the adenovirus major late promoter and the SV40 enhancer. We selected a clone for further work which we call C3H-BMP2. To facilitate the localization of engrafted cells, we further infected C3H-BMP2 with the retrovirus BAGa bearing the *lacZ* gene that encodes for β-galactosidase (β-gal); thus our geneticallyengineered cell line co-expressed *lacZ* as a marker gene and the gene for the therapeutic protein rhBMP-2. We confirmed double immunofluorescence that both proteins were being synthesized by C3H-BMP-2. Only β-gal, and not rhBMP-2, was found in BAGα infected wildtype C3H10T1/2 (C3H-WT) cells. The non-progenitor cell line CHO (CHO-WT) were genetically engineered by transfecting with an rhBMP-2 construct encoding a bicistronic transcript of human BMP-2 and DHFR under the control of the adenovirus major late promoter.

Secretion of rhBMP-2 was measured in the conditioned medium in which the cells had been grown. As determined by *in vitro* bioassay of conditioned medium, C3H-BMP2 cells secreted BMP-2 protein at the rate of 5+/-2.3 ng/24hrs/10⁷ cells and CHO-BMP2 secreted BMP-2 protein at the significantly higher rate of 841 +/- 88 ng/24 hrs/10⁷ cells. Bioassay and immunohistochemical assays revealed that C3H-WT secreted no rhBMP-2 protein. As expected, as measured by alkaline phosphatase expression, after 12 days in culture. C3H-BMP2 cells differentiated spontaneously into an osteoblastic lineage. In contrast, C3H-WT cells differentiated only when 50 ug/ml ascorbic acid and 100ng/ml rhBMP-2 ware added to the culture medium. Neither CHO-WT nor CHO-BMP2 differentiated under any circumstances.

### Enhanced bone repair by genetically engineered progenitor cells

To compare the *in vivo* therapeutic potential of C3H-BMP2 cells with that of other clones, we mounted 10° cells from each of the three clones on individual collagen sponges which were then transplanted individually into 2.5mm segmental defects in the radius of syngeneic mice (C3H/HeN). As a control, one group of mice received collagen sponges without any cell aliquot. For a further control, a fifth group of mice received a collagen sponge that carried no cells but did carry 3ug rhBMP-2. Mice were immunosuppressed by injections of 50 mg/kg/day Cyclosporine A, for 14 days. The healing process was monitored by periodic (every two weeks) x-ray photographs over a period of 8 weeks. X-ray analysis revealed a healing process in the radii of mice that received genetically engineered cells, with the highest rate (p<0.05) of bone callus formation in radii transplanted with C3H-BMP-2. At both 6 and 8 weeks after transplantation, the rate of callus formation in the C3H-BMP2 group surpassed that of the CHO-BMP2 group, even though CHO-BMP-2 express 168-fold more BMP-2 protein. Within two weeks after transplantation, increase in healing scores for mice transplanted with C3H-BMP2 or with CHO-BMP2 was 3-4 fold higher than for those transplanted with C3H-WT cells or collagen sponges without any cells; this difference was 1.8-2.4 fold at 8 weeks after transplantation. Compared to the CHO-BMP2 group, the scores for the C3H-BMP2 group were increased by 32% and 20%, at 6 and at 8 weeks after transplantation, respectively (P<0.05). Callus formation in mice that had received C3H-WT cells did not differ statistically from that in mice into that had received collagen sponges carrying no cells at all. We observed similar and even more pronounced trends when we compared the ability of the various cell line transplants to induce bone formation, as analyzed by mineral deposition and the size of the area of calcified tissue.

At four weeks after transplantation, the increase in mineral deposition scores of C3H-BMP2 cells as measured by relative fluorescence density was nine-fold greater than that of C3H-WT and 11-fold greater than mice into which had been transplanted collagen sponges not carrying any cells or protein; at eight weeks after transplantation these differences were four- and eight-fold, respectively. When compared to the genetically engineered non-progenitor cells CHO-BMP2, the increase in mineral deposition scores of C3H-BMP2 cell transplants were 140% and 165% at four and eight weeks after transplantation, respectively.

Also at four weeks after transplantation, histomorphometric analysis of the transplant areas revealed that the size of new calcified tissue area formed by C3H-BMP2 cell transplants was 2.0-2.8-fold larger than that of C3H-WT cell transplants or of the transplants of collagen sponges carrying no cells and no protein: at eight weeks after transplantation this difference was 3-fold. At eight weeks after transplantation the size of the calcified tissue area in which C3H-BMP2 cells had been engrafted was 163% greater than those in which CHO-BMP2 cells had been engrafted.

Bone formation is one of the reflections of bone repair. With only slight variation, evaluation of the parameters of bone formation clearly revealed that both of the cell lines that expressed rhBMP-2 had a marked ability to enhance bone regeneration. The ability to enhance bone regeneration of the genetically engineered progenitor cells, C3H-BMP2, was higher than that of genetically engineered non-progenitor cells, CHO-BMP2. Note that the *in vitro* secretion level of rhBMP-2 detected for C3H-BMP2 cells was 168 times less than that of CHO-BMP2 cells.

### Regeneration patterns displayed by genetically engineered progenitor cells

During the healing process, histological analysis of the fracture area revealed heterogenous morphological structures in the various transplantation groups. Four weeks after transplantation, in non-union radial sites into which were transplanted C3H-BMP2 cells, we observed a unique regeneration process which included well organized new growth of bone and cartilage within the boundaries of the fracture edges. In addition, a collar of differentiating and calcifying chondrocytes was formed around the original edge of the bone defect. Another important characteristic of areas to which C3H-BMP2 was transplanted was the formation of *de novo* bone not linked to the bone defect edges. At eight weeks after transplantation, there were no signs of bone resorption activity in any of the various parts of the transplantation sites. In all of the other experimental groups, including the group that received collagen sponges carrying rhBMP-2 protein, any bone or cartilage observed was formed in a disorganized manner.

The CHO-BMP2 transplants exhibited new disorganized cartilage and bone formation and relatively extensive new ectopic bone formation around the edges of the bone defect with no signs of any organized structure. Such ectopic bone formation in the muscles surrounding the transplant area was not observed in any except the CHO-BMP2 transplants In contrast to the C3H-BMP2 transplants, eight weeks after CHO-BMP2 transplantation we observed resorption of ectopic bone.

Transplants of C3H-WT cells and of collagen sponges carrying no cells or protein exhibited very mild responses which were expressed as minimal *de novo* bone formation on bone defect edges, formation of disorganized cartilage around the defect edges, and a relatively low number of hypertrophic and calcifying chondrocytes.

Comparison between transplantations of collagen sponges carrying either C3H-BMP2 or 3ug rhBMP-2 revealed that introduction of the pure protein did enhance the formation of *de novo* trabecular bone and cartilage. However, in this case the *de novo* bone and cartilage did not form in alignment to the original defect edge cortices, which are easily distinguished from the new trabecular bone.

In summary, the effects of transplanted C3H-BMP2 cells differed from that of other groups (including rhBMP-2 protein) not only in efficiency, but also in the nature of the healing process. Following C3H-BMP2 transplantation, bone and cartilage formed around the fracture edge appeared organized and oriented according to the original pattern of radial bone, thus better reconstructing its original structure. C3H-BMP-2 cell transplants also induced *de novo* bone formation unrelated to the defect edge. In all groups the formation of new cartilage and bone appeared to a certain extent concentrated on the defect edges. CHO-BMP2 cells and rhBMP-2 induced cartilage and bone formation that appears to be lacking any organization and orientation and did not follow the normal configuration of the healing process.

### Engraftment and cell fate of genetically engineered progenitor cells.

Cells infected with the BAGα retrovirus carry *lacZ* to permit easy identification of the cells *in vivo*. Two weeks after transplantation, transplanted clones C3H-BMP2 and C3H-WT were observed localized along the transplantation site, creating cell layers at the bone defect edges. These cell layers surrounded the defect edges in an organized manner. Morphologically, most transplanted cells resembled fibroblasts, and some resembled chondrocytes.

Four weeks after transplantation, C3H-BMP2 cells were found as lining cells in newly formed bone trabecules, displaying osteoblastic morphology. Double immunofluorescence assays revealed the co-expression of β-gal and rhBMP-2 in these cells. Unlike the C3H-BMP2 cells, C3H-WT cells displayed mainly a fibroblastic morphology, and were incorporated into the connective tissue formed in the transplant area and in the bone defect edges. Relatively few C3H-WT cells were localized to newly formed bone tissue, as was found with C3H-BMP2 cells. The same pattern of engraftment of transplanted C3H-BMP2 and C3H-WT was identified at 6 and 8 weeks after transplantation, although β-gal positive cells were reduced in number shown. These observations demonstrated that progenitor cells can engraft successfully and survive at least up to eight weeks in a regenerating bone site. Moreover, they can localize successfully at specific areas in the regenerated bone, differentiate, and incorporate to host tissues. Our data indicated that C3H-BMP2 have a tendency towards osteogenic differentiation and integration into osteogenic tissue, while C3H-WT cells have a tendency towards differentiation and integration into connective tissue.

The non-progenitor cells were CHO cells that do not differentiate into the osteogenic pathway, and which had previously been shown to survive in progenitor tissue (subcutaneous area) for as long as four weeks. Here we have shown that genetically engineered progenitor C3H-BMP2 cells survived transplantation, engrafted, and differentiated to form regenerated bone sites. Furthermore, progenitor cells were significantly more advantageous in their therapeutic potential than were similarly treated non-progenitor genetically engineered cells CHO-BMP2.

In our cell mediated gene therapy model we observed both the autocrine mechanism and the paracrine mechanism. Both *in vitro* and *in vivo*, both the genetically engineered progenitor cell line C3H-BMP2 and the genetically engineered non-progenitor cell line CHO-BMP2 expressed and secreted rhBMP-2. Therefore both of these lines exhibit the paracrine mechanism. However, in addition. C3H-BMP2 cells exhibit the autocrine mechanism, and also probably respond to signal molecules expressed by local host cells and matrix proteins. In contrast to these two genetically engineered celllines. C3H-WT cells cannot be controlled by either a paracrine or an autocrine mechanism, but probably respond to some extent to signal molecules secreted by adjacent host cells in the transplantation area. This is similar to effects reported in the CNS. Evidence of autocrine activity was demonstrated in *vitro* by the ability of C3H-BMP2 cells to differentiate spontaneously into osteogenic cells, while C3H-WT cells differentiated only following the application of exogenous rhBMP-2. *In vivo*, after transplantation, engraftment, and differentiation. C3H-BMP2 cells had the morphological appearance of osteoblasts and were found to integrate mainly into new bone and cartilage tissues. C3H-WT cells, on the other hand, had the morphological appearance of fibroblasts and were found to integrate mainly into the connective tissue surrounding the transplantation site. These results indicate that C3H-WT cells are less capable of differentiating into osteogenic cells and bone tissue, because they lack expression of the transgene rhBMP-2. We concluded that by themselves, progenitor cells can engraft, but that the genetically engineered progenitor cells can both engraft and differentiate along the osteogenic pathway. We have concluded that the expression of rhBMP-2 in C3H-BMP2 cells can induce osteogenic differentiation *in vitro* as well as *in vivo*, thus directing the differentiation pattern of the transplanted cells from the fibroblastic to the osteogenic pathway. The ability of progenitor cells to localize specifically within two weeks after transplantation, and furthermore to surround the defect edges, indicates that progenitor cells are probably susceptible to local signals from neighboring cells, which can affect their localization and engraftment. In the case of genetically engineered progenitor cells, there is in addition their reaction to the autocrine mechanism in which they respond to their own signal molecules.

By histological examination we found that cartilage and bone was formed around the defect edges only in the C3H-BMP2 transplants. We believe that it is the specific localization and orientation of the transplanted C3H-BMP2 cells at the defect edges that is responsible for orderly formation. Although high doses of rhBMP-2 have been reported to heal large bone defects, the new bone formed did not appear to have normal structure, nor did it appear to be a continuation of the original bone. This is in contrast to the bone formation induced by rhBMP-2 delivered through expression of the transgene in genetically engineered transplanted cells. Thus, it appears that the use of progenitor cell mediated gene therapy for tissue repair would be more advantageous than the use of other therapies.

Our analysis of bone formation parameters in regenerating bone sites revealed that C3H-BMP2 cell transplants were superior to other experimental groups (including CHO-BMP2 cell transplants) in promoting new bone formation. We suggest that the combined paracrine and autocrine mechanisms achieved by C3H-BMP2 cells, and not only the paracrine mechanism of secreted rhBMP-2, are responsible for the pronounced therapeutic potential of these cells. In support of these observations is the fact that although C3H-BMP2 cells secrete 168 times less rhBMP-2 than do CHO-BMP2 cells (*in vitro* and assuming that this difference is kept *in vivo*), C3H-BMP2 cell transplants formed bone surpassing CHO-BMP2 transplants. Since *in vivo* the effect of the local application of rhBMP-2 is dose dependent, we concluded that in addition to the paracrine mechanism, the therapeutic effect of the presence of the C3H-BMP2 cells was driven by the autocrine mechanism and also perhaps by signaling effects from neighboring host cells.

Progenitor cells are currently being used for the repair of damage in the central nervous system (CNS). In such systems, progenitor cells and have been found to differentiate and become engrafted into the host tissue. Originally, it was hoped that neural progenitor cells could be genetically engineered to exert a therapeutic effect by their ability to respond to local factors (including the transgene), differentiate, and become an integral component of the host tissue, as well as to have the ability of the transgene to produce a paracrine mechanism itself. However, in this context, genetically engineered neural progenitor cells in the CNS have not yet proved to be superior to genetically engineered non-progenitor cell types. This is in contrast to the present results which demonstrated that the engineered progenitor cells (C3H-BMP2) are in fact superior to the engineered non-progenitor cells (CHO-BMP2).

The use of BMP's for gene therapy of non union bone fractures was reported previously in two different approaches. The first approach used direct BMP-4 gene delivery (by plasmid on matrix) to femoral segmental defect in rats. The authors hypothesized that the healing observed by direct plasmid delivery is due to uptake of the plasmid and expression of BMP-4 by fibroblastic cells migrating to the damage site. According to this approach, expressed BMP-4 exhibits paracrine mechanism/effects on osteogenic cells, but is not likely to exhibit both paracrine and autocrine mechanisms. In addition, it is more likely to achieve high levels of transgene expression utilizing ex vivo gene delivery, compared to direct gene delivery (due to low transduction rate using direct plasmid delivery). The second approach , like this report uses cell mediated gene therapy for the delivery of rhBMP-2 into femoral segmental defects in rats. For this purpose the authors used W-20-17 cells, a murine stromal cell line, which were genetically engineered to express rhBMP-2. and were shown to induce bone healing upon local transplantation to the fracture site. Although W-20-17 cells differentiate in the osteogenic pathway in response to rhBMP-2, the authors attributed the healing effect mainly to the delivery of rhBMP-2 (paracrine mechanism) by these cells. It is not known whether genetically engineered W-20-17 cells differentiate *in vitro,* moreover their fate *in vivo* is not determined yet. Our results, however, demonstrate that genetically engineered progenitors expressing rhBMP-2 have both paracrine and autocrine effects, when combined together produce an increased healing effect surpassing that genetically engineered cells which have a paracrine effect only.

RhBMP-2 have many effects on different cell types and tissues, and is therefore suitable for gene therapy to other organs, beside bone. Recently, rhBMP-2 was found to have an inhibitory effect on smooth muscle proliferation *in vitro and in vivo*. In this study, direct infection of injured carotid artery in rats with recombinant adenovirus encoding human BMP-2, inhibited smooth muscle cells proliferation and prevented the thickening of the intima layer of the injured artery.

In our model system for gene therapy based on genetically engineered progenitor cells we have achieved several goals: I) the *de novo* bone formation is continuous with the existing bone in non-union fractures; ii) the biological efficiency of this system is high enough that it works even when the concentration of the transgene product is low; iii) It is known that the half-life of rhBMP-2 is very short; by creating a system in which rhBMP-2 is continuously expressed, we subject the cells to the continued presence of the rhBMP-2 protein: iv) Our system is simple, simple to use, and appropriate for use in human beings. In this regard, we are currently conducting experiments to test the possibility that human progenitor cells can be used in our model system. This model is also appropriate for the therapeutic intervention for healing lesions intissues or organs other than bone.

Among the many possible therapies for tissue lesion are protein therapy and various styles of gene therapy. While it seems that our model for genetically engineered cell mediated gene therapy is probably more efficient than protein therapy, we have not yet compared our system with systems in which adenoviruses or plasmids are used as the vehicle for the delivery of rhBMP-2 gene into bone defects. We shall address these questions experimentally in the near future.

### METHODS

### Construction of genetically engineered cell lines

C3H-BMP2 cells were generated from the pluripotent cell line C3H10T1/2 as described previously. In the presence of 8mg/ml polybrene, the selected clone (T5/C3H-BMP2) was infected with the BAGα retrovirus bearing the *lacZ* gene that codes for β-gal. Wild type C3H10T1/2 cells were also infected with the BAG-α retrovirus, in order to generate a C3H-WT cell line expressing β-gal. Both cell lines were selected with 0.5mg/ml of the antibiotic G418. CHO-BMP2 were generated as described previously.

### In vitro characterization of geneticallvengineered cell lines

Secretion of rhBMP-2 by the genetically engineered cell lines C3H-BMP2 and CHO-BMP2 was determined by bioassay as described previously. W-20-17 cells were cultured with conditioned medium obtained from each of the cell lines for 24 hours. Parallel cultures of W-20-17 cells were cultured with increasing concentrations of rhBMP-2 protein. Twenty-four hours after the addition of the rhBMP-2 protein and conditioned medium, alkaline phosphatase activity was determined in the W-20-17 cell lysate by incubation with 50mM glycine, 0.05% Triton X-100, 4 mM MgCl2 and 5 mM p-nitrophenol phosphate, pH 10.3, at 37°C for 30 min. and measuring spectrophotometric absorbance at 405nm. Secretion of rhBMP-2 in conditioned medium from each experimental cell line was assessed by comparing alkaline phosphatase activity in W-20-17 cell lysates (incubated with the conditioned media) to a standard curve generated from the alkaline phosphatase activity of W-20-17 cells incubated with increasing concentrations of rhBMP-2 as described above.

Co-expression of β-galactosidase and BMP-2 was demonstrated by double-immunofluorescence. The *in vitro* differentiation phenotypes were determined by culturing the progenitor cell lines C3H-BMP2 and C3H-WT in varying plating densities for 12-19 days, and by using the following histochemical staining procedures: alkaline phosphatase histochemical staining (Sigma kit 86-R) as an early marker for osteoblastic differentiation, alcian blue to define chondroblasts and Oil red Ostaining to define adipocytes.

### Double immunofluorescence

Double immunofluorescence in frozen sections was used to demonstrate the *in vivo* co-expression of β-gal and BMP-2 in C3H-BMP2 cells. Cells were fixed with methanol acetone (1/1 by volume). The mixture of antibodies were prepared as follows: primary antibodies of monoclonal mouse IgG2b anti-β-gal at a concentration of 20 ug/ml, and polyclonal rabbit anti-rhBMP-2-R230 or -W8 (1:100 dilution) directed against the mature region of human BMP-2. Fixed cells and the antibody mixtures were incubated at room temperature for 1hr. Incubation with the mixture of primary antibodies was followed by incubation with biotinylated goat anti-mouse IgG2b Ab, followed by streptavidin Cy3 and finally by goat anti-rabbit antibody-FITC conjugated (1:80 dilution) Jackson 111-015-003), each incubation for 30 min at room temperature.

### In vivo transplantation

Before being transplanted *in vivo,* cells were trypsinized and counted with a Coulter®-21 counter. Aliquots of 10⁶ cells were mounted on individual type I collagen sponges (Collastat®, 2mmX2mmX4mm. Vitaphore Corp.) and transplanted into C3H/HeN mice, into a standard 2.5 mm gap created in the right radius. In all, there were five experimental groups: a collagen sponge carrying: I) an aliquot of 10⁶ C3H-BMP2 cells; ii) an aliquot of 10⁶ genetically engineered non-progenitor cells (CHO-BMP2); iii) an aliquot of 10⁶ progenitor cells which had not been genetically engineered (C3H-WT); iv) no cells at all; v) no cells but on which we placed 3ug pure rhBVIP-2. As experimental host animals. 3-4 mo old female C3H/HeN mice were used. These mice were immunosupressed with injections of 1mg/mouse/day CyclosporineA (Sandoz) from day 0 over a period of 2 weeks. The transplantation procedure took place immediately after this 2 week period. Transplantations also began at day 0.

### X-ray analysis

At days 0, 2, 4, 6 and 8 weeks after transplantation of the collagen sponges X-ray photographs were taken of each mouse. The X-rays were scanned into a computer, and measurements were done using the NIH image program 1.66. For each time point, defect healing was determined by calculating the optical density ratio which is equal to the mean optical density value of the gap (original size, as measured by X-ray for each mouse on day 0) divided by the mean optical density of the ulna.

### Mineral deposition analysis

To assess the amount of mineral deposition in the transplantation areas, mice were labeled with the fluorescent mineralization marker calcein green. Mice were injected with the 2.5mg/kg dye i.p. 7 and 2 days before sacrifice. Mice were sacrificed at four and eight weeks after transplantation. Samples of the operated limbs were fixed in ethanol (70% and subsequently 80% and 100%) and were embedded into plastic blocks (Immuno Bed Polysciences). Fluorescence labels were observed on 7um thick sections, using a fluorescent microscope supplied with an FITC filter. The relative fluorescence density was calculated as the total fluorescence density measured in the gap area (the original size of the gap for each mouse on day 0), divided by the total fluorescence density of a constant area of the ulnar cortex. Measurements were done using the NIH image program 1.66.

### Histomorphometry and Histology

For histology and histomorphometry 7um plastic sections were stained with Masson Trichrom and H&E stains. Total calcified tissue area in the gap (original size of each mouse on day 0), was measured using automatic image morphometry analysis (Galai:CUE-3 Electro Optical Inspection and Diagnostic Laboratories Ltd. MigdalHaemek, Israel).

### In vivo detection of genetically engineered progenitor cells

Detection of engrafted C3H-BMP2 and C3H-WT cells *in vivo* required the sacrifice of the mice at 2, 4, 6 and 8 weeks after transplantation. Operated limbs were fixed in 4% paraformaldehyde (PFA) for 1 hour after transcardial perfusion with 10 ml of 4% PFA, cryoprotected with 5% sucrose overnight, embedded, and frozen 15 um sections were prepared with in a cryostat(Bright, model OTF). The engrafted cells and their progeny were detected by X-gal histochemical staining. First they were fixed in a solution of 0.25% glutaraldehyde, 0.1M Na Phosphate (PH. 8.3), 5mM EDTA and 2mM MgCl₂ for 30min. Then the cells were washed three times in a solution of 0.1M Na-Phosphate, 2mM MgCl2, 0.1% deoxycholate, 0.2% Nonident P.40. Finally, the cells were stained by incubating them in a solution of 1mg/ml X-gal, 5mM K3Fe(CN)6, 5mM K4Fe(CN)6.3H2O,0.1M Na-Phosphate, 2mM MgCl2, 0.1% deoxycholate, 0.2% Nonident P.40, at room temperature (in the dark) overnight. Co-expression of the genes for β-gal and BMP-2 in C3H-BMP2 cells was revealed by double-immunofluorescence (as described above).

### EXAMPLE 12 Systemic extraskeletal effects of rhBMP-2.

RhBMP-2 administered systemically (20 days) affects various extraskeletal organs in osteopenic old mice.

### Methods:

### Muscle strength measurements:

Muscle strength was measured by the Grip Test which determines the ability of the mouse to grip a horizontally fixed wire, and the speed with which it does so, measured in seconds.

### Histomorphometry, histology, and histochemical staining for ALP activity

Mice internal organs (liver, kidney, testis, and spleen) were dissected, fixed in 4% buffered formalin and embedded in paraffin. Femurs were dissected and fixed in 4% buffered formalin, decalcified, embedded in paraffin. 5mm sections were stained for H&E. Histochemical staining of the cells for alkaline phosphatase (ALP) activity was carried out by using a Sigma kit (No. 86R). The areas of ALP positive colonies were measured in each 35 mm dish using automatic image morphometric analysis (Glai).

### MSCs proliferation detected by BrdU

Marrow Stromal Stem Cells (MSCs) were cultured on chamber slides. Cell culture medium was removed and replaced with the diluted BrdU labeling solution. Following 2 hour incubation at 37°C. cells were immunohistochemically stained by using Zymed BrdU staining kit according to manufacturer's directions (Zymed Laboratories Inc., South San Francisco, USA). Briefly, cells were fixed with 70%-80% alcohol for 30 min at 4°C, blocked for endogenous peroxidase activity with 3% hydrogen peroxide in methanol for 10 min, treated with denaturing solution for 30 min for DNA denaturation. Following treatment with PBS, containing 10% non-immune goat serum for 10 min at room temperature (to minimize the nonspecific binding of reagents in subsequent steps), the cells were incubated with biotinylated mouse anti-BrdU antibody for 60 min at room temperature, and streptavidin conjugated with horseradish peroxidase for 10 min at room temperature. Each step was terminated by three washes with PBS. Specifically bound antibodies were visualized by using 3,3'-diamino benzine (DAB) mixture. All slides were counterstained with hematoxylin solution. Results were expressed as percent of positive cells (brown nuclei) of total cells.

### Apoptosis of MSCs

Apoptotic cells were detected by a TUNEL kit according to manufacturer's protocol (Oncor). For quantitative analysis of apoptotic cells, random 4-7 fields of each well in chamber slides were observed and apoptotic and total cells were counted on microscope through a 20x or 40x objective lens in the fluorescent mode. The percentage of apoptotic nuclei was calculated for each field and the data were expressed as means for each chamber slide.

### RNA isolation and RT-PCR

RNA isolation was performed using RNAzol B (Biotecx Lab. Inc., Texas. USA) according to the manufacturer's protocol. Briefly, brains were homogenized in the reagent using a glass-Teflon homogenizer. MSCs were collected by trypsin, and cell pellets were homogenized by RNAzol B. Homogenate was mixed with chloroform and centrifuged, which yielded the top aqueous phase, interphase and the bottom organic phase. RNA was precipitated from the aqueous phase by the addition of isopropanol, washed and dissolved in water. RT-PCR was performed with modifications of procedure as described previously (4), by using 2ug of total RNA.

### Results and discussion:

### Section 1: Extra-skeletal effects of systemic administration of rhBMP-2 in oldBALB/c osteoporotic male mice.

### BMP-2 treatment increases muscle strength in old mice

BMP-2 had significant effect on muscle strength similar in both doses 0.5 and 1.0 ug/day. Treated old mice were able to grip on the wire and position themselves on it, with legs and tail in shorter time, compared to nontreated controls. Control non-treated mice were not able to grip the wire horizontally, because of decrease in muscle strength.

### Systemic effects of BMP-2 on testicular structure and function.

rhBMP-2 ( 0.5; 1 and 5 ug/day for 20 days) stimulated spermatogenesis. There was an increased number of germ cells in treated animals. Quantitative analysis of spermatogenesis revealed significant increase in germ cell number in spermatogenic tubuli in treated mice, with the highest effect of the dose 1 and 5 ug. An increased number of germ cells in seminiferous tubules followed systemic treatment with BMP-2. and correlated well with a significant decrease in the number of apoptotic germ cells (TUNEL) found in treated mice, when compared to nontreated controls (P<0.05). These results indicated that systemic administration of rhBMP-2 to old mice increased muscle strength, and stimulated testicular germ cell proliferation and differentiation. This finding is consistent with data obtained by Zhao et al., who described BMP-8 as critical for testicular function and development. In general, the role of BMP-2 in spermatogenesis is still poorly understood. Future studies we will be needed to clarify the biological mechanisms involved in enhanced spermatogenesis and muscle strength caused by systemic administration of rhBMP-2.

### The in vitro effects of rhBMP-2 on MSCs, obtained from old BALB/c mice rhBMP-2 increases ALP activity in vitro of MSCs obtained from old mice

MSCs colonies (obtained from old mice) were treated in vitro with rhBMP-2 at doses of 0.1. 0.5 ,1.0 and 5.0 ug/ml, for 8 days. Size of alkaline phosphatase (ALP) positive MSCs colonies significantly increased at all doses, except 0.1 ug/ml. These results indicate the direct effect of rhBMP-2 on MSCs obtained from osteoporotic old mice, and supports the result that systemic administration has beneficial effects on osteoporosis in mice through the stimulation of MSCs.

### BMP receptors in brains obtained from old osteoporotic mice

Systemic administration of rhBMP-2 to old osteoporotic mice, significantly increased the expression of BMP receptors IA and II in their brains. Experimental design included 4 groups: young control, old control, old treated with 0.5 ug/day/mouse rhBMP-2 for 20 days, old treated with 1.0 ug/day/mouse rhBMP-2, for 20 days. RNA isolation from brains was performed by using RNAzol B (Biotecx Lab. Inc., Texas, USA) according to the manufacturer's protocol. RT-PCR was performed with modifications, by using 2 ug total RNA. The BMP receptor primers were a kind gift from Dr. J. Lauber and G. Gross (GBF, Germany), designed according to their cDNA sequences. RT-PCR quantitative results were expressed by normalizing the densitometry units of BMP receptors to RPL19 (internal control). Systemic treatment of rhBMP-2 upregulated expression of BMPR-II (in brains of mice treated systemically with 0.5 and 1.0 ug rhBMP-2) and BMPR-IA (1.0 ug rhBMP-2). Brains obtained from old mice express significantly lower levels of BMPR-IA and BMPR-II mRNA, when compared to young mice. We showed previously (unpublished data) that old mice did not have as good memory as young mice (as determined in Water Maze test), and according to the present invention BMP-2 might have beneficial effects on memory in old mice.

### RhBMP-2 administered systemically reverses bone loss in Post-menopausal (type I) osteoporosis in ovariectomized mice.

Recombinant human BMP-2 induced local cartilage and bone formation *in vivo*. In addition. BMP-2 stimulated osteoblastic phenotype expression in osteogenic cell lines. Our preliminary results indicated that E2 administered *in vitro*, upregulated BMP-2 gene expression in MSCs obtained from both, sham and OVX-operated mice. These results indicated that BMP-2 was one of E2's target genes, and might be responsible for E2's anabolic effect in OVX mice. Based on these data, we hypothesized that systemic administration of rhBMP-2 to OVX mice, might reverse their bone loss (anabolic effect). OVX mice were randomly divided three groups, and were all treated systemically for 20 days (i.p injections); control mice (injected 200 ul PBS/BSA daily, n=8); mice systemically treated with 1 ug/day/mouse (n=8); and mice systemically treated with 5 ug/day/mouse (n=8). Body weight had not significantly changed during the 20 days of injection. Internal organs, spleen, liver and kidney, were dissected from all mice, fixed in 4% buffered formalin and embedded in paraffin, 5 um sections were stained for H&E. There were no signs of toxicity and/or fibrosis in mice systemically injected with 1 and 5 ug of rhBMP-2. Femurs of controls and OVX-treated mice were dissected, fixed in 4% buffered formalin, decalcified, embedded in paraffin, and 5um sections were stained for H&E. Systemic administration of rhBMP-2 (1 and 5 ug/day) stimulated trabecular bone formation in femoral bones.

These results support our initial hypothesis that systemically administered rhBMP-2 is capable of reversing osteopenia in the femural bones of osteoporotic ovariectomized mice. After 20 days of systemic treatment by rhBMP-2, mice bone marrow was cultured, and stromal cells (MSCs) were isolated in 4 wells chamber slides, for 12 days. Systemic treatment with rhBMP-2 significantly decreased apoptosis of MSCs and increased MSCs proliferation, indicating that the systemic anabolic effect of rhBMP-2 on OVX mice, occurred through stimulation of MSCs obtained from OVX mice. This mechanism is similar to the mechanism we described in the case of senile osteoporotic mice systemically treated with rhBMP-2.

### EXAMPLE 13: Encapsulation of genetically engineered pluripotent mesenchymal cells conditionally (tet-regulated) expressing rhBMP-2

We explore here the possibility of using tet-regulated rhBMP-2 expression in C3H10T1/2 cells as a delivery vehicle for rhBMP-2 (paracrine mechanism only), without engraftment of the cells into host tissue (autocrine and paracrine effects). Cell encapsulation, as has been described previously (Hortelano, 1996), separates physically the host environment and immune system from transplanted cells, but allows diffusion of rhBMP-2 into the host environment.

### Methods

**Encapsulation and transplantation of capsules:** Cell encapsulation was performed as described previously (Chang, 1994; Hortelano, 1996). Briefly, a suspension of cells was mixed with 2.5% potassium alginate in a syringe and extruded with a syringe pump through a 27G needle at the rate of 39.3 ml/h. An air jet concentric to the needle created fine droplets of the cell/alginate mixture that were collected in a CaCl₂ solution. Upon contact, the droplets gelled. The outer alginate layer was chemically cross linked with poly-L-lysinhydrobromide for 6 minutes and then with another layer of alginate. Finally, the remaining free alginate core was dissolved with sodium citrate for 6 minutes to yield microcapsules with an alginate-PLL-alginate membrane containing cells. Capsules were maintained *in vitro* prior to transplantation *in vivo*, with DMEM supplemented with 2mM L-glutamine, 10% fetal calf serum and penicillin/streptomycin 100 units/ml.

Using a 10ml syringe and a 19G needle, approximately 5ml of tightly packed capsules in PBS were injected into a subcutaneous area in the back of old and young BALB/c mice. Mice were given drinking water with or without the addition of 0.5 mg/ml DOX. Upon sacrifice, some capsules were retrieved back *in vitro* and the rest of the transplant area was evaluated for histology using paraffin sections and H&E staining.

### Results:

### Encapsulating genetically engineered progenitor cells for a controlled rhBMP-2 protein delivery system

C9 cells were encapsulated in rounded alginate capsules as described previously (Chang 1994; Hortelano, 1996). Approximately 5ml of capsules were transplanted sub-cutaneously into the backs of two old and two young BALB/c mice (in each group one mouse was treated with DOX and the other mouse was not). After 27 days mice were sacrificed and analyzed. In DOX treated mice no signs of bone or cartilage tissue formation were found, either in young or in old mice. In mice that were not treated with Dox, bone and cartilage formation could be seen surrounding the capsules transplant area on macroscopic as well as in histological sections. In young recipients, the bone formed around the capsules was significantly more prominent than the bone formed in old recipients. This same pattern was observed inside the capsules, where chondrogenic differentiation occurred; however, the pattern was seen on a lower scale in the old recipients. These results suggest that the differentiation of the cells inside the capsules might be affected by some unknown factors from the "host"environment (reciprocal mechanism).

### Conclusions

Our results indicate that genetically engineered progenitors, as a controlled delivery system of rhBMP-2, with cell encapsulation, could be an elegant solution. Cell encapsulation enables the complete separation of the encapsulated cells from the host environment and protects them from the immune system (Chang 1994; Hortelano 1996). Upon transplantation of the capsules into sub-cutaneous area, bone formation was under DOX control. Treatment of the mice with DOX inhibited bone and cartilage formation that was observed in non-treated mice. Such a controlled delivery system can be used to deliver rhBMP-2 gene and other genes locally or systemically, as in diseases like osteoporosis and osteoarthritis.

In our model encapsulation also allows compartmentalization of different components of the reciprocal differentiation model. The paracrine mechanism observed outside the capsules, the autocrine mechanism inside the capsules, and the reciprocal mechanism is observed by the different effects of the host environment (young mice or old mice) on the differentiation of the cells inside the capsules. Our unpublished data shows that, genetically engineered mesenchymal cells conditionally expressing rhBMP-2 can engraft and differentiate in vivo, enhance bone formation in ectopic sites and bone repair in non-union fractures. Our results here also show that these cells can be encapsulated and can serve as a protein delivery system for rhBMP-2 or other gene products, systemically or locally, without engraftment of the transplanted cells to the host tissue.

### EXAMPLE 14: Regional gene therapy for bone utilizing Ad-BMP-2 (Adenovirus carrying BMP-2 cDNA)

Recombinant human Bone Morphogenetic Protein 2 (rhBMP-2), a member of the TGF-β superfamily, is a highly osteoinductive agent that can induce bone formation in ectopic sites like regenerating bone. In vitro, rhBMP-2 has been shown to induce the osteogenic differentiation of mesenchymal cell lines and of marrow derived stromal cells. Moreover, overexpression of rhBMP-2 induces the in vitro differentiation of the mesenchymal cell line C3H10T1/2.

Marrow stromal cells (MSCs) are pluripotent mesenchymal cells that also serve as precursors for osteoprogenitors cells, which are the main cellular mediators for bone formation. In vitro and in the presence of a number of supplements such as β-glycerophosphate, ascorbic acid and dexamethasone, MSCs can differentiate into osteoblasts. In addition, several cytokines including BMP-2 can induce osteoblastic differentiation of MSCs. When transplanted into ectopic sites. MSCs have been shown to induce in vivo bone formation.

MSCs in general, and human MSCs in particular, have been seriously considered as vehicles for cell therapy and for gene therapy. As vehicles for cell therapy MSCs have mainly been considered for use in healing cartilage and bone defects or disorders like osteogenesis imperfecta. As vehicles for gene therapy, MSCs have been transduced in vitro to express genes (human factor IX and growth hormone) so as to deliver these transgenes systemically, by expressing the gene in the bone marrow environment. It has been suggested that MSCs could be genetically engineered for the treatment of bone -related diseases like osteogenesis imperfecta and osteoporosis.

MSCs have also been shown to be effectively transduced with adenoviral vectors and retroviral vectors. In this study we explored the possibility of increasing the osteogenic potential of MSCs in vitro and in vivo by rhBMP-2 gene transfer, using adenoviral vector. In addition, we planned to monitor the effects of rhBMP-2 expression on differentiation, proliferation and apopiosis in vitro and on ectopic bone formation in vivo. Finally we explored the possibility of introducing Adeno-BMP-2 directly in vivo, in order to establish "direct" gene therapy for bone regeneration.

### MATERIALS AND METHODS

Animals: BALB/c male mice age 6-7 weeks were used for harvesting MSCs and for in vivo transplantations. Cell Culture: Bone marrow stromal cells (MSCs) wereharvested as described previously (Gazit et al., 1998). Briefly, MSCs were isolated from the femurs and tibias of young (6-7 weeks) BALB/c mice. The epiphyses of the dissected bones were removed and content of the bone marrow cavity was expelled under the hydrostatic pressure using tissue culture medium delivered into the marrow space by a syringe with a 22G needle. The bone marrow cells thus obtained were resuspended in tissue culture mediumfollowing passages through 19G, 21G, and 23G needles: the cells were counted and cultured for 12 days in MEM-a supplemented with 10% FCS. Pen-Strep 100 U/ml, 2mM glutamine and supplemented with 50mg/ml ascorbic acid. 10mM -Glycerophosphate and 10-8 M dexamethasone. The marrow cells were plated into 35mm dishes (Nunc) and four well chamber-slides (Nunc), at a density of 1.25x105 cells/cm2. On day 6, these cultures were infected with adeno-BMP2 and adeno-lacZ (10pfu/plated cell = m.o.i.=100 (multiplicity of infection=pfu/cell),at 37°C for two hours. Analysis for differentiation, proliferation, and apoptosis was done on days two, six, and 14after infection. C3H10T1/2 cells were grown in DMEM supplemented with 2mM L-glutamine, 100units/ml penicillin, 100units/ml streptomycin, and 10% FCS. Cells were infected at 20 m.o.i at 70% confluency. Expression of BMP-2 in infected cells was demonstrated by immunohistochemistry 48 hours after infection. Adenovirus preparation and Infections: Recombinant Adeno-BMP-2 virus (Ad.5 sub360, E1 and partial E3 regions deleted; Logan and Shenk. 1984) was prepared by inserting human BMP-2 cDNA Eco R1 fragment into the Eco RV site in the Ad5 linker, in reverse orientation. The resulting plasmid was cut with NotI and ligated back in the opposite orientation resulting in the correct orientation for BMP-2. The expression of human BMP-2 was driven by the CMV promoter. The recombinant adenovirus was generated by infecting 293 cells with the described construct and analyzing selected clones with Southern blot analysis.

Recombinant adeno-lacZ (E1 and partial E3 regions deleted:) was a gift from the Genetics Institute. Cambridge, MA. The expression of β-galactosidase (β-gal) was driven from the CMV promoter. RNA isolation and RT-PCR: Total RNA was isolated using RNAzol B (Biotecx Lab. Inc., Texas. USA)according to the manufacturer's protocol. Briefly, MSC were collected by trypsin, the cell pellets were homogenized by RNAzol B. The homogenate was mixed with chloroform and centrifuged, which yielded the top aqueous phase, the interphase, and the bottom organic phase. RNA was precipitated from the aqueous phase by the addition of isopropanol, washed and dissolved in water. RNA was also extracted by RNeasy Mini Kit (QIAGEN Inc., CA. USA).

RT-PCR was performed as described previously (Orly et al., 1994) but with 2mg total mRNA. BMP-2 primers were designed based on themurine human BMP-2 cDNA sequence (Wozney et al., 1988). For the 492 bp human BMP-2 band, we used primers as follows: the forward primer: 5'-CATCCCAGCCCTCTGAC-3'
the reverse primer: 5'-CTTTCCCACCTGCTTGCA-3'.
The internal control RPL19 was designed as described previously (Orly et al., 1994). W20 bioassay for the detection of BMP-2: To assess the secretion of active rhBMP-2, MSCs were cultured asdescribed above and incubated with complete DMEM medium supplemented with 100mg/ml heparin (Sigma H3393)(conditioned medium). Medium was collected after 24 hours, four days post infection with the adenoviral constructs. The W20 bioassay was performed as described previously (Thies. 1992). Briefly, W-20-17 cells were cultured with conditioned medium obtained from each of the cell lines for 24 hours. Parallel cultures of W-20-17 cells werecultured with increasing concentrations of rhBMP-2 protein. Twenty-four hours after the addition of the rhBMP-2 protein and conditioned medium, alkaline phosphatase activity was determined in the W-20-17 cell lysate by incubation with 50mM glycine, 0.05% Triton X-100. 4 mM MgCl2 and 5 mM p-nitrophenol phosphate, pH 10.3, at 37°C for 30 min. and measuring spectrophotometric absorbance at 405nm. Secretion of rhBMP-2 in conditioned medium from each experimental cell line was assessed by comparing alkaline phosphatase activity in W-20-17 cell lysates (incubated with the conditioned media) to a standard curve generated from the alkaline phosphatase activity of W-20-17 cells incubated with increasing concentrations of rhBMP-2 asdescribed above. Immunohistochemistry for the detection of BMP-2: Cells were fixed with methanol acetone and immunohistochemistry was done using a standard kit (Zymed kit 95-9943). We used primary polyclonal antibodies. 1:100 dilution of rabbit anti-rhBMP-2, W8, R230 (Israel et al., 1992) or 20 mg/ml 17.8.1 monoclonal antibody: these mixtures were incubated at room temperature for one hour. Our negative control for polyclonal antibodies was normal rabbit serum; our control for monoclonal antibody was mouse IgG (monoclonal universal negative control - Immunostain). Detection of β-galactosidase by histochemical staining: After two hours incubation in 4% paraformaldehyde, histochemical staining for X-gal was done by fixing the cells or whole tissue sample for 30 min in a solution of 0.25% glutaraldehyde, 0.1M Na Phosphate (PH. 8.3), 5mM EGTA and 2mM MgCl₂. Cells were then washed 3 times with a solution of 0.1M Na Phosphate.2mM MaCl2, 0.1% deoxycholate. 0.2% Nonident P.40). Finally, the cells were stained by incubation in a solution of 1mg/ml X-gal, 5mM K3Fe(CN)6, 5mMK4Fe(CN)6-3H2O, 0.1M Na Phosphate. 2mM MgCl2, 0.1% deoxycholate, and 0.2% Nonident P.40, in the dark at room temperature overnight. Assays for measuring differentiation, proliferation, and apoptosis. Alkaline phosphatase expression: For testing differentiation cultures were assayed for alkaline phosphatase (ALP) expression. Histochemical staining of MSC colonies for ALP activity was carried out by using a Sigma kit (No. 86R). The colony number per dish was counted using a microscope, and the areas of ALP positive colonies percent were measured in each 35mm dish using automatic image morphometric analysis (ComputerizedMorphometric System, Galai. Israel).
BrdU staining: MSCs were cultured on chamber slides (Nunk);cell culture medium was removed and replaced with the diluted BrdU labeling solution. After a two hour incubation at 37°C. cells were immunohistochemically stained using Zymed BrdU staining kit according to manufacturer's directions (Zymed Laboratories Inc.. South SanFrancisco. USA). Results were expressed as percent of the total number of cells that had brown nuclei (positive cells).
Apoptosis: Culture medium was replaced by PBS. and cells were stained with 10 mg/ml propidium iodide (PI) (Pandey and Wang 1995). Cells that contained highly dense nuclear chromatin with irregular inclusions were defined as apoptotic. In cells that were not apoptotic the DNA stained moderately and homogeneously throughout the entire nucleus (Keren-Tal et al.. 1995). For a positive control we used MSCs that we treated with 100mg/ml etoposide (Smeyne etal., 1993) for 6hr.

For quantitative analysis we randomly chose four to seven microscopic fields of each well, using a 20x or a 40x objective lens in fluorescent mode. We counted the number of total cells and among them the number of apoptotic cells. The percentage of apoptotic nuclei was calculated for each field and the data were expressed as means for each chamber slide (Keren-Tal et al., 1995). In vivo transplantation and histological analysis: For in vivo transplantations. MSCs were cultured under the conditions described above. After two weeks in culture, cells were trypsinized and plated at a concentration of 1.6x105 cells/well on vitrogen collagen gels in 24 well plates (according to manufacturer instruction. Vitrogen 100R. Collagen Corporation, USA). Twenty-four hours after plating on vitrogen, the MSCs were infected with either adeno-BMP-2or adeno-lacZ constructs (10-pfu/plated cell). Twenty-four hours after infection, the collagen gels containing the cells were removed from plate and transplanted into the sub-cutaneous area of young (6-8 weeks old) male BALB/c mice; this is called a syngeneic transplantation. Mice were sacrificed at 10 or at 20 days aftertransplantation. In another assay, MSCs were doubleinfected with adeno-BMP-2 and adeno-LacZ (each at m.o.i.=100) and transplanted into Sprague-Dawley rats subcutaneosly in the abdomen and sacrificed after 7 or 20 days.

For direct delivery of adeno-BMP-2 in vivo, a viral suspension of 3 x 109 pfu's of recombinant adeno BMP-2 and 3 x 109 pfu's of adeno-lacZ were mounted on collagen sponges (CholestatR, Vitaphore Corporation. 2mmX2mmX4mm size), and delivered directly into the abdominal subcutaneous tissue of BALB/c mice. Mice were sacrificed on days 10 and or 20 after transplantation. Samples were evaluated by embedding them in paraffin and staining 7um sections with H&E. Transplants of adeno-lacZ were processed by whole mount X-gal histochemical stain.followed by histological evaluation.

### RESULTS

### In vitro Characterization

As determined by the percent of β-galactosidase (β-gal) positive cells from total infected cells, the infection of MSCs by adeno-lacZ was found to be highly efficient (over 90%). Four days after infection the secretion of rhBMP-2 was determined by bioassay (see Methods), and was found to be three times higher in adeno-BMP-2 infected cultures than in control cultures. The secretion levels were 22 +/-2.57ng/24 hours/10⁶ cells in adeno-BMP-2 infected cultures compared to 8 +/-0.74 ng/24hours/10⁶ cells in control cultures. The expression that we detected in non-infected cultures was due to the endogenous expression of murine BMP genes. BMP-2 expression was observed using immunohistochemistry two days after infection by adeno-BMP-2 and RT-PCR four days after infection with adeno-BMP-2. In addition to that, expression of BMP-2 was also detected in C3H10T1/2 cell line infected with Ad-BMP-2, 48 hours after infection. Interesting findings were observed regarding differentiation, proliferation, and apoptosis of MSCs infected with adeno-BMP-2. Differentiation (determined by ALP expression) was found to increase significantly as a function of time after infection, and the absolute values were higher in cultures infected with adeno-BMP-2 than in the controls on two, six, and 14 days post infection. Proliferation was measured by BrdU staining: the percent of cells positive for BrdU was significantly higher in adeno-BMP-2 infected cells than in the controls on two, six, and 14 days post infection. Apoptosis was measured by PI staining: in the case of apoptosis our results were in opposition to those that we found for differentiation and proliferation. The fraction of cells that were apoptotic in adeno-BMP-2 infected cultures was less than that in control cultures. In both experimental and control cultures the percent of apoptotic cells decreased with time, indicating that BMP-2 enhances differentiation and proliferation, and inhibits apopstosis in MSCs infected with Adeno-BMP-2.

### In vivo Ectopic Bone Formation

MSCs grown on vitrogen and infected with adeno-BMP-2, or with adeno-lacZ as a control, were transplanted into a subcutaneous area in in the abdomen area of male BALB/c mice. Ten days after transplantation the beginning of bone mineralized tissue could be observed in MSCs infected with adeno BMP-2 transplant, and an increased number of blood vessels was noted in transplantation area (compared to controls). In contrast, mineralized tissue was not found in mice transplanted with MSCs infected with adeno-lacZ. Twenty days post transplantation of MSCs infected with adeno-BMP-2, bone and blood vessels formation were observed in the transplantation area.

Murine MSCs (BALB/c) double infected with adeno-BMP-2 and adeno-lacZ were transplanted into Sprague-Dawley rats. Seven days post transplantation. β-gal positive cells were detected in the rat tissue with no signs for inflammatory cells. Twenty days after transplantation, mineralized tissue was found in the transplantation area. These results indicate that MSCs from a different species than the host animal (Heterogeneic transplantation) can survive and induce bone formation in the host tissue. Ectopic bone Formed by direct adeno BMP-2 delivery: Induced bone formation was observed twenty days after an aliquot of 3x10⁹ pfu's of adeno-BMP-2 were delivered directly to subcutaneous tissueon a collagen sponge matrix. No signs of bone formation were observed following the direct delivery of adeno-lacZ (3x10⁹ pfu's) to subcutaneous tissue. 10 days post transplantation. However, we did observe β-gal positive muscle cells around the transplant area, indicating the efficiency of the adenoviral vectors to transduce cells in subcutaneous tissue in vivo.

### DISCUSSION

Recombinant human BMP-2 is known to induce bone formation in vivo (Wozney et al., 1988; Wang et al., 1990; Volek-Smith and Urist, 1996), and to promote osteogenic differentiation of mesenchymal and marrow stromal cell lines in vitro (Katagiri et al., 1990;Chen, et al., 1991; Thies et al., 1992;Rosen et al., 1994; Chudhari et al., 1997; Yamaguchi et al., 1995;Yamaguchi et al.. 1996; Hughes et al., 1995) as well as of primary cultures (Rickard et al., 1994;Puleo et al., 1997; Hanada et al., 1997; Balk et al., 1997). Moreover, it has been shown that overexpression of rhBMP-2 in the C3H10T1/2 mesenchymal cell line induced osteogenic differentiation of these cells (Ahrens et al., 1993; Wang et al., 1993; Gazit et al. 1997). Our results indicate that rhBMP-2 protein enhances osteoblastic differentiation in MSCs both in vitro and in vivo, either by accelerating the differentiation of committed cells or by committing non committed cells to differentiate in the osteoblastic pathway (unpublished data).

Our findings also indicate that the presence of the rhBMP-2 protein alters the in vitro cellular parameters of MSCs, including differentiation, proliferation, and apoptosis (unpublished data). Based on these findings, we hypothesiszed that efficiently infecting MSCs with adeno-BMP-2 might cause an effect similar to that described for BMP-2 protein. We found that in MSCs infected with Ad-BMP-2 cellular differentiation and proliferation increased but apoptosis was reduced. It is interesting to note that in such adeno-BMP-2 infected MSCs, the differentiation rate increased and apoptosis rate decreased in cultures which were kept longer. Since we hypothesize that apoptosis occurs mainly in cells that have not differentiated, it is possible that the reduction in apoptosis was an indirect outcome of the increase in differentiation. Since proliferation takes place at an early stage of differentiation (Shukunami et al 1998), a similar mechanism might explain the increase in the MSC proliferation rate. In control cultures we also observed an increase in differentiation and a reduction in apoptosis, probably due to the expression of endogenous murine BMP gene (as detected in bioassay). However, infecting these control cultures with an adenoviral vector encoding human BMP-2 caused a three fold increase in the expression of rhBMP-2, as detected by our bioassay. Since we found that MSCs can be efficiently transduced with an adenoviral vector, these genetically engineered cells become capable of inducing bone formation in vivo. Thus they can be used as an inducers in gene therapy for healing bone lesions. The induction in vivo of bone formation by transduced MSCs is expected to occur not only by paracrine mechanism of the expressed rhBMP-2 on host cells, but also by the autocrine mechanism of the transgene on the MSCs themselves. By this autocrine mechanism, they are induced to differentiate and can thus form bone themselves (Reciprocal Differentiation system, Gazit et al., 1997). Indeed, our in vivo results demonstrate that MSCs infected with adeno-BMP-2 can induce bone formation in ectopic subcutaneous sites. Moreover, mouse MSCs infected with adeno-BMP-2 could survive and form bone in rats without any evidence of immune reaction even when the rats had not been immunosuppressed. These results indicate that genetically engineered MSCs have a significant osteogenic potential even in different species without eliciting immune response to the cells or the viral vector. The direct delivery of adeno-hBMP-2 was also found to have osteogenic effect in vivo, indicating that the adeno-BMP2 construct can penetrate into host tissue efficiently and express rhBMP-2 there. These findings open a new avenue in bone gene therapy. Efficient transduction of MSCs was reported before with retroviral (Li et al., 1995) and adenoviral vectors (Foley, 1997; Balk, 1997). Although high efficiencies of infection by retroviruses have been reported (Li et al., 1995; Chuah et al., 1998), generally such efficiency rates are low, significantly lower then the rates of infection with adenoviral vector. Unlike retroviruses, the expression of adenoviral vectors is short lived since they do not integrate into the genome of the infectedcells. Thus, adenoviruses are considered safer for therapeutic use than are retroviruses (Roemer and Friedmann, 1992; Kozarsky and Willson, 1993). This would be advantageous when transient and controlled biological activity of infected cells is desirable. As described in the literature, the use of transduced MSCs for gene therapy has focused mainly on the paracrine delivery of proteins for systemic effects, as described for hemophilia models (Lozier et al., 1994; Gordon et al., 1997; Hurwitz et al., 1997; Chuah et al., 1998), or for cytokine production affecting the hemopoetic environment (Allay et al., 1997; Foley et al., 1997). The suggestion that transduced MSCs can be used to treat bone diseases (Balk et al., 1997; Prockop. 1997) is confirmed by our data that show that adeno-hBMP-2 gene transduction of MSC's increases their osteogenic differentiation in vitro and bone formation in vivo. We conclude that MSC's can be efficiently transduced with a humanBMP-2 encoding adenoviral vector. Both the in vitro and the in vivo osteogenic potential of such transduced cells is increased. These results support the future use of such a system for ex vivo gene therapy for healing bone diseases. Our findings indicate that adenoviral vectors carrying rhBMP-2 cDNA can efficiently infect host cells in vivo, and thus enhance the local expression ofrhBMP-2.

### EXAMPLE 15: TRANSPLANTATIONS OF GENETICALLY ENGINEERED C3H10T1/2 CELLS (i.m.10/20 days), WITH COLLAGEN SPONGE CARRIER. IN C3H MICE.

### OBJECTIVE:

Chondroblastic and osteoblastic differentiation *in vitro* and enchondral bone formation *in vivo* are directedby and contingent on signaling cascades triggered by Bone Morphogenetic Proteins (BMPs) and their receptors. BMPs are members of the transforming growth factor-β superfamily. BMPs are known to promote the differentiation of pluripotent progenitor stem cells into cartilage and bone. C3H10T1/2 is a pluripotent progenitor cell line that can initiate the osteogenic and/or chondrogenic pathways upon the exogenous addition or recombinant expression of various BMPs. BMP-2, for which two type I receptors were characterized [BMPR-IA (Alk3) and BMPR-IB (Alk6)], and a type II receptor, (BMPR-II), have been identified. It is not clear whether both BMPR-IA and BMPR-IB are necessary to mediate the onset and progression of cellular differentiation in the direction of cartilage and/or bone formation, or ifone of them is sufficient.

Our preliminary data showed that C3H10T1/2 cells expressing recombinant BMP-2 differentiate into chondroblasts and osteoblasts *in vitro*. Here we shall characterize differentiation *in vivo* of C3H10T1/2 cells expressing different types of receptors, using the ectopic cell transplants in C3H mice.

Defining such signalling mechanisms could pave the way for the design of new therapeutic modalities in transplantation of genetically engineered C3H10T1/2 cells for gene therapy aimed at endochondral bone formation, or cartilage formation in fractures, osteoporosis, and osteoarthritis.

### CELL LINES

1. C3H10T1/2-BMP2: (C3H10T1/2 cell line over expressing hBMP2);
2. c3H-PTHR: (C3H10T1/2 cell line overexpressing PTH receptor);
3. C3H-BMP2-PTHR: (C3H10T1/2 cell line overexpressing both hBMP2 and PTH receptors);
4. C3H-dominant negative (dn) Alk 3 (Type I A receptor)-BMP2 (C3H10T1/2-LacZ construct over expressing rhBMP2, and dominant negative Type I A receptor;
5. C3H- dominant negative (dn) Alk 6 (Type I B receptor)-BMP2 (C3H10T1/2 - Lac-Z construct overexpresing rhBMP2, and dominant negative -Type I B receptor.

### RATIONALE:

The aim of this study is to determine the effects *in vivo* of the above genetic alterations made in C3H surface receptors, and to define the mechanism that determines cartilage and/or bone formation. Assuming that the different cell lines are committed to different differentiation pathways, which should reflect their *in vivo* differentiation pattern, and should lead to new modalities in cell-mediated gene therapy.

### METHODS:

### Cell lines and Culture Conditions:

The BMP-2, PTHR and BMP2-PTHR clones expressing rhBMP-2. PTHR and both rhBMP-2 and PTHR genes, respectively, were isolated and selected from C3H10T1/2 cells which had been transfected by plasmids that encodes hBMP-2 and rat PTHR (BMP2-PTHR was transfected twice). In both cases, gene transcription is driven from the LTR seqence of the myeloproliferative sarcoma virus (MPSV). Control or BMP-transfected C3H10T1/2 cells were selected by cotransfection with a plasmid mediating resistance againstpuromycin (5 ug/ml). C3H10T1/2 cells transfected with the rat PTHR were selected by cotransfection with the plasmid-mediating resistance against G418 (750 ug/ml).

Cells were grown in DMEM supplemented with L-glutamine (2mM).penicillin and streptomycin (100 units/ml) and 10% FCS.

### Transplantation Procedure:

For transplantation *in vivo* cell lines were grown in culture to confluency for one to two weeks (same conditions as mentioned above). After two weeks cells were trypsinized, harvested, counted and approximately 2.5-3.0 x 10⁶ cells were mounted on precut sterile collagen sponges "Collastat®" (size: 3mmx 3mm x 2mm), which were used to deliver the cells into the intramuscular transplantation site (rectal abdominal muscle) of C3H/HeN female mice, age6-8 weeks.

Animals were anaesthetized (2% Xylazine and 8.5% Ketamine, injected i.p.), and the cells mounted on the collagen sponge placed into the formed intramuscular pocket for the period of 10 or 20 days.

### Histological Analysis and Histochemistry:

Mice were sacrificed on day 10 and 20 after transplantations. Transplant and associated tissues were recovered, fixed in 4% formaldehyde, decalcified with De-cal solution (National Diagnostic, Atlanta, GA) overnight at room temperature and embedded in paraffin, using standard technique. 7-10 um sections were cut and mounted on slides and stained with H&E.

For X-gal histochemistry whole tissue samples were fixed in 4% paraformaldehyde, and processed according standard X-gal histochemistry protocol, embedded in paraffin and 20-30 um sections were cut and mounted on slides. The sections were counterstained with Nuclear Fast Red (NFR) to detect for β-Gal positive blue cells.

### RESULTS:

Morphological evaluation of the implanted tissues was done by analyzing for the formation of cartilage and/or bone in proximal and distal sites to the collagen sponge.

| C3H10T1/2-BMP2: | | |
|---|---|---|
| Day-20: | Bony ossicle with hypertrophic cartilage (HC), bone (B) and bone marrow (BM) | Sponge filled with proliferating cartilage. |

| PTHR: | | |
|---|---|---|
| Day-10 & 20: | No cells and tissue formation. | Connective tissue (CT) only |

| BMP2-PTHR: | | |
|---|---|---|
| Day-10: | No cells and tissue formation. | Connective tissue and cartilage (C). |
| | | |
| Day-20: | No cells and tissue formation. | Connective tissue and cartilage filling the collagen sponge |

| dn Alk 3 (Dominant Negative LA =Alk 3)-BMP2 (LacZ): | | |
|---|---|---|
| Day-10: | No cells and tissue formation. | Connective tissue only. |
| | | |
| Day-20: | No cells and tissue formation. | Connective tissue and cartilage |

| dnAlk 6 (Dominant Negative IB= Alk 6)-BMP-2 (lacZ): | | |
|---|---|---|
| Day-10: | Cartilage (C), bone mineral ized particles (BP) and note: β-gal (+) positive cells lining the BP. | Connective tissue(CT) only, |
| | | |
| Day-20: | Hypertrophic cartilage (HC) Cartilage bone mineralized particles | bonemineralized particles (BP). |

### CONCLUSIONS:

Our results clearly indicate that altering cells surface receptors in pluripotent progenitor cells, like C3H10T1/2, has a major efffect on differentiation pathways of these clones *in vivo.* Expression of dominant negative Alk6 (IB) receptors directed the *in vivo* differentiation towards bone and cartilage formation. In contrast, dominant negative Alk3 expression resulted in cartilage formation. Overexpression of PTH receptors resulted in cartilage formation as well (*in vivo*). Our results demonstrate that type IB and IA BMP2 receptors transmit different signals via genetically engineered progenitors and play critical role in osteogenic differentiation and thus can be used for cell mediated gene therapy for bone and/or cartilage diseases like osteoathritis and osteoporosis.

The foregoing descriptions detail presently preferred embodiments of the present invention. Numerous modifications and variations in practice thereof are expected to occur to those skilled in the art upon consideration of these descriptions. Those modifications and variations are part of the present invention, and believed to be encompassed within the claims appended hereto.

## Claims

1. A method for producing cells for implantation at the site of a bone infirmity in a human, said method comprising the steps of
(a) transforming a cultured human mesenchymal stem cell with a DNA encoding bone morphogenetic protein 2 (BMP-2); and
(b) culturing the human mesenchymal stem cell transformed in step (a), whereby the produced cells express BMP-2 and are suitable for implantation at the site of a bone infirmity in a human.

2. The method of claim 1, wherein said cultured human mesenchymal stem cell is a mesenchymal stem cell line.

3. The method of claim 1, wherein said cultured human mesenchymal stem cell is a primary cell.

4. The method of claim 1, wherein said cultured human mesenchymal stem cell expresses an endogeous BMP receptor.

5. A method for producing cells for implantation at the site of a bone infirmity in a human, said method comprising the steps of
(a) transforming a cultured human mesenchymal stem cell with a DNA encoding bone morphogenetic protein 2 (BMP-2) and a DNA encoding a BMP receptor; and
(b) culturing the human mesenchymal stem cell transformed in step (a), whereby the produced cells express BMP-2 and said BMP receptor, and are suitable for implantation at the site of a bone infirmity in a human.

6. The method of claim 5, wherein said cultured human mesenchymal stem cell is a mesenchymal stem cell line.

7. The method of claim 5, wherein said cultured human mesenchymal stem cell is a primary cell.

8. The method of claim 5, wherein said cultured human mesenchymal stem cell expresses an endogenous BMP receptor.

## Patentansprüche

1. Verfahren zur Herstellung von Zellen zur Implantation an der Stelle einer Knochenschwäche in einen Menschen, wobei das Verfahren folgende Schritte umfasst:
(a) Transformieren einer kultivierten humanen mesenchymalen Stammzelle mit einer DNA, welche Knochenwachstumsfaktor 2 (bone morphogenetic protein 2, BMP-2) kodiert; und
(b) Kultivieren der humanen mesenchymalen Stammzelle, die in Schritt (a) transformiert wird, wobei die hergestellten Zellen BMP-2 exprimieren und zur Implantation an der Stelle einer Knochenschwäche in einen Menschen geeignet sind.

2. Verfahren gemäß Anspruch 1, wobei die kultivierte humane mesenchymale Stammzelle eine mesenchymale Stammzelllinie ist.

3. Verfahren gemäß Anspruch 1, wobei die kultivierte humane mesenchymale Stammzelle eine Primärzelle ist.

4. Verfahren gemäß Anspruch 1, wobei die kultivierte humane mesenchymale Stammzelle einen endogenen BMP-Rezeptor exprimiert.

5. Verfahren zur Herstellung von Zellen zur Implantation an der Stelle einer Knochenschwäche in einen Menschen, wobei das Verfahren folgende Schritte umfasst:
(a) Transformieren einer kultivierten humanen mesenchymalen Stammzelle mit einer DNA, welche Knochenwachstumsfaktor 2 (bone morphogenetic protein 2, BMP-2) kodiert und einer DNA, welche einen BMP-Rezeptor kodiert; und
(b) Kultivieren der humanen mesenchymalen Stammzelle, die in Schritt (a) transformiert wird, wobei die hergestellten Zellen BMP-2 und den BMP-Rezeptor exprimieren, und zur Implantation an der Stelle einer Knochenschwäche in einen Menschen geeignet sind.

6. Verfahren gemäß Anspruch 5, wobei die kultivierte humane mesenchymale Stammzelle eine mesenchymale Stammzelllinie ist.

7. Verfahren gemäß Anspruch 5, wobei die kultivierte humane mesenchymale Stammzelle eine Primärzelle ist.

8. Verfahren gemäß Anspruch 5, wobei die kultivierte humane mesenchymale Stammzelle einen endogenen BMP-Rezeptor exprimiert.

## Revendications

1. Procédé à manufacturer des cellules pour les implanter au point d'une débilité d'os dans un homme, comprenant les étapes suivantes:
(a) transformer une cellule souche cultivée, humaine, mesenchymale avec un ADN encodant des proteins morphogenetiques d'os 2 (BMP-2), et
(b) cultiver la cellule souche humaine, mesenchymale, transformée dans l'étape (a), éxprimante BMP-2 et apte pour être implanter au point d'une débilité d'os dans un homme.

2. Procédé selon la revendication 1, dans lequel la cellule souche cultivée, humaine, mesenchymale est une ligne de cellule souche mesenchymale.

3. Procédé selon la revendication 1, dans lequel la cellule souche cultivée, humaine, mesenchymale est une ligne de cellule primale.

4. Procédé selon la revendication 1, dans lequel la cellule souche cultivée, humaine, mesenchymale exprime un recepteur de BMP endogène.

5. Procédé à manufacturer des cellules pour les implanter au point d'une débilité d'os dans un homme, comprenant les étapes suivantes:
(a) transformer une cellule souche cultivée, humaine, mesenchymale avec un ADN encodant des proteins morphogenetiques d'os 2 (BMP-2) et un ADN encodant un recepteur de BMP, et
(b) cultiver la cellule souche humaine, mesenchymale, transformée dans l'étape (a), éxprimante BMP-2 et le recepteur de BMP, et apte pour être implanter au point d'une débilité d'os dans un homme.

6. Procédé selon la revendication 5, dans lequel la cellule souche cultivée, humaine, mesenchymale est une ligne de cellule souche mesenchymale.

7. Procédé selon la revendication 5, dans lequel la cellule souche cultivée, humaine, mesenchymale est une ligne de cellule primale.

8. Procédé selon la revendication 5, dans lequel la cellule souche cultivée, humaine, mesenchymale exprimet un recepteur de BMP endogène.
